# EUROPEAN PATENT APPLICATION

(11) **EP 0 855 387 A1**
(43) Date of publication of application: **29.07.1998**
(21) Application number: 96928682.2
(22) Date of filing: 29.08.1996
(51) Int. Cl.: C07C 237/42, A61K 31/165

(54) **2-ACYLAMINOBENZAMIDE DERIVATIVES AND PREVENTIVE AND REMEDY FOR DISEASES CAUSED BY THE SUPERMULTIPLICATION OF VASCULAR INTIMAL CELLS**

(30) Priority: 07.09.1995 JP 267629/95; 27.12.1995 JP 354952/95; 18.01.1996 JP 37035/96
(71) Applicant: KISSEI PHARMACEUTICAL CO., LTD., Matsumoto-City Nagano-Pref. 399 (JP)
(72) Inventor: HARADA, Hiromu, Nagano 399-74 (JP); KUSAMA, Hiroshi, Nagano 399 (JP); NONAKA, Yoshinori Fureguransu-Hanamizuki C-202, Minamiazumi-gun Nagano 399-82 (JP); KAMATA, Koji Fureguransu-Merodi A-101, Nagano 390 (JP); HOTEI, Yukihiko Kopo-Yamaji 205, Nagano 399-83 (JP); IYOBE, Akira Medio-Hotaka A-101, Nagano 399-83 (JP); FUJIKURA, Hideki Sanraifu-Inukai A-202, Nagano 390 (JP); SATOH, Fumiyasu, Nagano 390 (JP)
(74) Representative: Baverstock, Michael George Douglas
(86) International application number: JP9602415
(87) International publication number: WO9709301

(57) **Abstract**

The present invention relates to 2-acylaminobenzamide derivatives represented by the general formula: 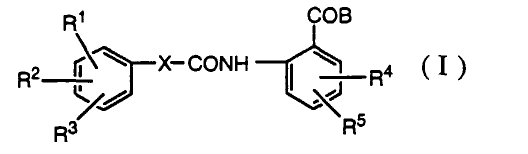 wherein R¹, R², R³, R⁴ and R⁵ represent each a hydrogen atom etc.; X represents a vinylene group etc.; B represents a group represented by the general formula:

-N(R⁶)(R⁷)

wherein R⁶ and R⁷ represent each a hydrogen atom etc., a group represented by the general formula:

-NH-(CH₂)ₙ-A-R⁸

wherein A represents a single bond etc.; R⁸ represents a hydroxy group etc. or a hydroxyamino group which are useful as agents for the prevention and treatment of diseases caused by excessive proliferation of vascular intimal cells.

## Description

### Technical Field

The present invention relates to 2-acylaminobenzamide derivatives which have an inhibitory effect on excessive proliferation of vascular intimal cells and are useful as medicaments.

### Background Art

In the event that a blood vessel happens to be stenosed due to some factors, the circulation of the blood is obstructed so as to cause an ischemic state. For example, the occurrence of this situation in the coronary arteries would result occasionally in ischemic diseases such as angina pectoris and myocardial infarction. At present, percutaneous transluminal coronary angioplasty (hereinafter referred to as PTCA), which involves recanalizing an atheromatous stenosed lesion in a coronary artery by means of a balloon catheter, directional coronary atherectomy (hereinafter referred to as DCA), which involves resecting selectively diseased tissues of a stenosed lesion, and the like are popular procedures for the treatment of diseases caused by such a blood vessel stenosis and have been clinically used for patients suffering from the diseases.

However, for example, vascular intima receives injury during the dilating operation by means of the balloon catheter in PTCA surgery and thereby vascular intimal cells proliferate excessively to heal the injured lesion, leading to narrowing of the arterial lumen. DCA and stent as well as PTCA injur vascular intima when operating or implanting and thereby vascular intimal cells proliferate excessively leading to narrowing of the arterial lumen. Lipid accumulation in blood vessels, vascular intimal cells proliferation and collagen accumulation occur due to some factors and then advance of atherosis leads the lesion to occlusion. Without any treatment, this stage may advance to a serious situation such as angina pectoris and myocardial infarction again.

At present, as an agent for the prevention and treatment of restenosis after PTCA surgery, 2-(3,4-dimethoxycinnamoylamino)benzoic acid (generic name: Tranilast) represented by the formula: 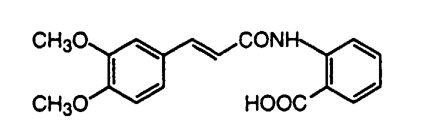 has been developed clinically. However, PTCA has problems that the incidence of restenosis after surgery is high, about 30-40%, and when administering Tranilast after surgery, about 10-15% restenosis was observed. Thus, although the incidence of restenosis is significantly reduced by the administration of Tranilast, more improvement has been desired. Furthermore, the daily dose of Tranilast is 600mg and is higher than that (300mg) for administration to patients suffering from allergic diseases. It is desirable also that Tranilast should be administered consecutively at least for 3-6 months after surgery because the risk of the incidence of restenosis is high during this term. Accordingly, Tranilast treatment is attended by troublesome administration control. In addition, Tranilast has the property of being easily converted into its dehydration ring-closure compound represented by the formula: 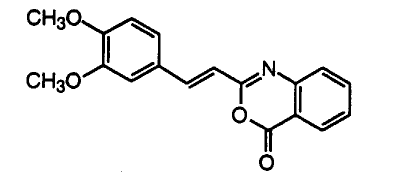 and the above dehydration ring-closure compound is easily produced as a by-product in the process of producing Tranilast and by heating Tranilast. The above dehydration ring-closure compound produced in such a process is difficult to remove by conventional methods such as recrystallization. As a consequence, it makes purification of Tranilast very difficult. Furthermore, recently, patients with stenosed coronary arteries have a tendency to increase due to change of eating habits, and PTCA, DCA and the like will go on increasing in operation frequency. Therefore, it has been desired to develop drugs having a better inhibitory effect on abnormal proliferation of vascular intimal cells and having a higher stability as described above.

### Disclosure of Invention

The present invention relates to a 2-acylaminobenzamide derivative represented by the general formula: 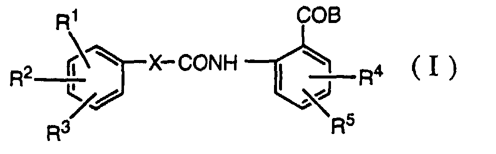 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which may be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X represents a vinylene group which may have a cyano group, an acetylene group, an ethylene group, a methylene group or a single bond; R⁴ and R⁵ are the same or different and each represents a hydrogen atom, a hydroxy group, a halogen atom, a lower alkyl group or a lower alkoxy group; B represents a group represented by the general formula:

-N(R⁶)(R⁷)

wherein R⁶ and R⁷ are the same or different and each represents a hydrogen atom, a lower alkyl group, a cycloalkylalkyl group or an aralkyl group a group represented by the general formula:

-NH-(CH₂)ₙ-A-R⁸-

wherein A represents a single bond, a group represented by the general formula:

-O-(CH₂)ₘ-

wherein m in an integer of from 2 to 6 or a group represented by the general formula:

-N(R⁹)(CH₂)ₚ-

wherein R⁹ represents a hydrogen atom or a lower alkyl group which may have a hydroxy group, an amino group or a mono or di(lower alkyl)amino group as a substituent; and p is an integer of from 2 to 6; R⁸ represents a hydroxy group or an amino group which may be mono- or di-substituted by lower alkyl groups; and n is an integer of from 2 to 6 or a hydroxyamino group and a pharmaceutically acceptable salt thereof.

The present invention relates to a pharmaceutical composition comprising the above 2-acylaminobenzamide derivative or pharmaceutically acceptable salt thereof.

The present invention relates to an agent for the prevention and treatment of diseases caused by excessive proliferation of vascular intimal cells which comprises, as an active ingredient, the above 2-acylaminobenzamide derivative or pharmaceutically acceptable salt thereof.

The present invention relates to a method for the prevention and treatment of diseases caused by excessive proliferation of vascular intimal cells which comprises administering the above 2-acylaminobenzamide derivative or pharmaceutically acceptable salt thereof.

The present invention relates to the use of the above 2-acylaminobenzamide derivative or pharmaceutically acceptable salt thereof for the manufacture of a pharmaceutical composition for the prevention and treatment of diseases caused by excessive proliferation of vascular intimal cells.

Furthermore, the present invention relates to the use of the above 2-acylaminobenzamide derivative or pharmaceutically acceptable salt thereof as an agent for the prevention and treatment of diseases caused by excessive proliferation of vascular intimal cells.

### Best Mode for Carrying Out the Invention

In the compounds represented by the above general formula (I) of the present invention, the term "lower alkyl group" means a straight- or branched-chain alkyl group having from 1 to 6 carbon atoms such as a methyl group, an ethyl group, a propyl group, an isopropyl group, a butyl group, an isobutyl group, a *sec*-butyl group, a *tert*-butyl group, a pentyl group, an isopentyl group, a neopentyl group, a *tert*-pentyl group and a hexyl group, and the term "lower alkoxy group" means a straight- or branched-chain alkoxy group having from 1 to 6 carbon atoms such as a methoxy group, an ethoxy group, a propoxy group, an isopropoxy group, a butoxy group, an isobutoxy group, a *sec*-butoxy group, a *tert*-butoxy group, a pentyloxy group, an isopentyloxy group, a neopentyloxy group, a *tert*-pentyloxy group and a hexyloxy group.

The term "lower alkoxycarbonyl group" means a straight- or branched-chain alkoxycarbonyl group having from 2 to 7 carbon atoms such as a methoxycarbonyl group, an ethoxycarbonyl group, a propoxycarbonyl group, an isopropoxycarbonyl group, a butoxycarbonyl group, an isobutoxycarbonyl group, a *sec*-butoxycarbonyl group, a *tert*-butoxycarbonyl group, a pentyloxycarbonyl group, an isopentyloxycarbonyl group, a neopentyloxycarbonyl group, a *tert*-pentyloxycarbonyl group and a hexyloxycarbonyl group.

The term "aralkyl group" means the above lower alkyl group substituted by an aromatic hydrocarbon group such as a phenyl group and a naphthyl group, and the term "aralkyloxy group" means the above lower alkoxy group substituted by the above aromatic hydrocarbon group, and the term "cycloalkyl group" means a 3- to 7-membered cyclic alkyl group, and the term "cycloalkylalkyl group" means the above lower alkyl group substituted by the above cycloalkyl group, and the term "cycloalkylalkoxy group" means the above lower alkoxy group substituted by the above cycloalkyl group.

The term "halogen atom" means a fluorine atom, a chlorine atom, a bromine atom and an iodine atom, and the term "lower acyl group" means an alkylcarbonyl group having from 2 to 7 carbon atoms, said alkyl being a straight- or branched-chain alkyl, such as an acetyl group, a propionyl group and a butyryl group. The term "lower alkylsulfonyl group" means an alkylsulfonyl group having from 1 to 6 carbon atoms, said alkyl being a straight- or branched-chain alkyl, such as a methanesulfonyl group and an ethanesulfonyl group.

The term "lower alkylene group containing oxygen atoms in the chain" means an alkylene group having from 1 to 6 carbon atoms and containing oxygen atoms in the chain such as a methylenedioxy group and an ethylenedioxy group.

Some of the 2-acylaminobenzamide derivatives represented by the above general formula (I) of the present invention and pharmaceutically acceptable salts thereof are known compounds and are described in the literature. The 2-acylaminobenzamide derivatives represented by the above general formula (I) of the present invention can be prepared by methods described in the literature (for example, Egypt. J. Chem., Vol .21, No.2, pp.115-131(1978); Egypt. J. Chem., Vol .28, No.3, pp.235-238(1985); Egypt. J. Chem., Vol .32, No.6, pp.651-660(1991); published Japanese patent applications (*kokai*) Nos.Sho.47-2927, Sho.61-36273, Sho.63-295543, Sho.63-295544 and Hei.1-26543; Indian J. Chem., Vol.13, No.4, pp.326-328(1975); Indian J. Chem., Vol.20B, No.5, pp.394-397(1981); Bull. Trav. Soc. Pharm. Lyon, Vol.17, No.4, pp.143-148(1973); U. A. R. Chem., Vol.13, No.4, pp.379-390(1970); J. Chem. U. A. R., Vol.12, No.1, pp.57-68(1969); U.S. patent No.3,192,214; published U.K. patent No.1,099,829; Synthesis, Vol.12, pp.977-979 (1981); J.Med. Chem., Vol.9, No.16, pp.809-812(1966); J. Med. Chem., Vol.12, No.1, pp.164-166(1969); Chem. Pharm. Bull., Vol.22, No.3, pp.623-627(1974); J. Chem. Soc., pp.4420-4421(1956); J. Chem. Soc. C, Vol.24, pp.2290-2295(1966); Rev. Roum. Chim., Vol.22, No.8, pp.1217-1223(1977); Rev. Roum. Chim., Vol.24, No.11-12, pp.1509-1520(1979)), analogous methods thereto or these methods combined with other known methods.

For example, the compounds of the present invention can be prepared by allowing a carboxylic acid derivative represented by the general formula: 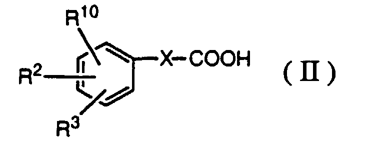 wherein R¹⁰ represents a hydrogen atom, a halogen atom, a protected hydroxy group, a lower alkyl group, a lower alkoxy group, a protected hydroxy lower alkoxy group, a cycloalkylalkoxy group, an aralkyloxy group, a lower acyl group, a nitro group, a cyano group, a protected amino group which may be mono-substituted by a lower alkyl group, an amino group di-substituted by lower alkyl groups, a lower alkoxycarbonyl group or a lower alkylsulfonyl group; and R², R³ and X have the same meanings as mentioned above or a reactive functional derivative thereof such as an acid halide and an activated ester to react with a 2-acylaminobenzamide derivative represented by the general formula: 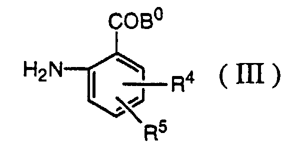 wherein B⁰ represents a group represented by the general formula:

-N(R⁶)(R⁷)

wherein R⁶ and R⁷ have the same meanings as mentioned above a group represented by the general formula:

-NH-(CH₂)ₙ-A⁰-R¹¹

wherein A⁰ represents a single bond, a group represented by the general formula:

-O-(CH₂)ₘ-

wherein m has the same meaning as mentioned above or a group represented by the general formula:

-N(R¹²)(CH₂)ₚ-

wherein R¹² represents a hydrogen atom or a lower alkyl group which may have a hydroxy group, a protected amino group, a protected amino group mono-substituted by a lower alkyl group or an amino group di-substituted by lower alkyl groups as a substituent; and p has the same meaning as mentioned above; R¹¹ represents a hydroxy group, a protected amino group which may be mono-substituted by a lower alkyl group or an amino group di-substituted by lower alkyl groups; and n has the same meaning as mentioned above or a hydroxyamino group; and R⁴ and R⁵ have the same meanings as mentioned above in the presence of base in the presence or absence of a dehydrating agent or a condensing agent in an inert solvent, and, if necessary, removing the protective group.

The compounds represented by the general formula (I) of the present invention can be prepared by allowing a compound represented by the general formula: 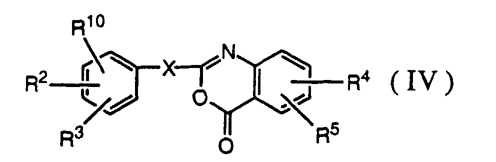 wherein R², R³, R⁴, R⁵, R¹⁰ and X have the same meanings as mentioned above to react with an amine compound represented by the general formula:

H-B⁰ (V)

wherein B⁰ has the same meaning as mentioned above in an inert solvent, and, if necessary, removing the protective group.

The compounds represented by the general formula (I) of the present invention can be prepared by allowing a 2-acylaminobenzoic acid derivative represented by the general formula: 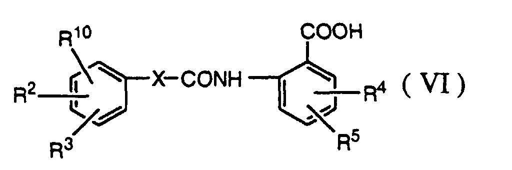 wherein R², R³, R⁴, R⁵, R¹⁰ and X have the same meanings as mentioned above or a reactive functional derivative to react with the amine compound represented by the above general formula (V) in the presence of a base in the presence or absence of a dehydrating agent or a condensing agent in an inert solvent, and, if necessary, removing the protective group.

For example, of the compounds represented by the above general formula (I) of the present invention, a compound represented by the general formula: 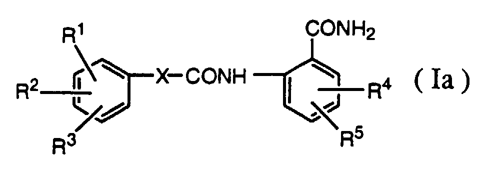 wherein R¹, R², R³, R⁴, R⁵ and X have the same meanings as mentioned above can be prepared by allowing a 2-acylaminobenzoic acid ester derivative represented by the general formula: 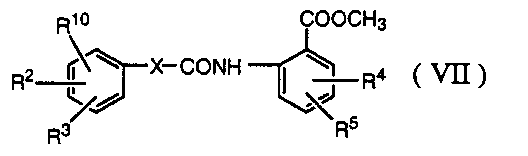 wherein R², R³, R⁴, R⁵, R¹⁰ and X have the same meanings as mentioned above to react with ammonia in methanol in the presence of a catalytic amount of sodium cyanide on heating in a sealed tube, and, if necessary, removing the protective group.

Of the compounds represented by the above general formula (I) of the present invention, a compound represented by the general formula: 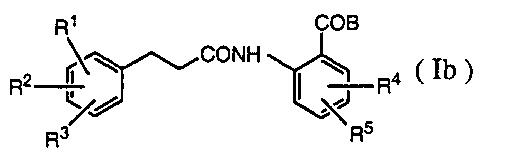 wherein B, R¹, R², R³, R⁴ and R⁵ have the same meanings as mentioned above can be prepared by reducing a compound represented by the general formula: 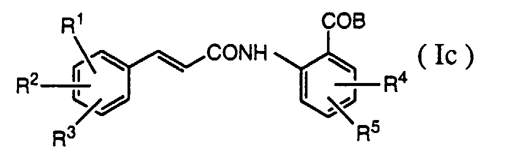 wherein B, R¹, R², R³, R⁴ and R⁵ have the same meanings as mentioned above using a catalyst such as palladium on carbon in a stream of hydrogen.

The compounds represented by the above general formulae (II) and (V) using as starting materials in the above production processes are available commercially or can be prepared by methods described in the literature or analogous methods thereto.

The compounds represented by the above general formula (III) using as starting materials in the above production process are available commercially or can be prepared by allowing an isatoic anhydride derivative represented by the general formula: 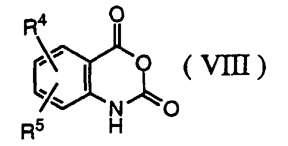 wherein R⁴ and R⁵ have the same meanings as mentioned above or a compound obtained by treating an anthranilic acid derivative represented by the general formula: 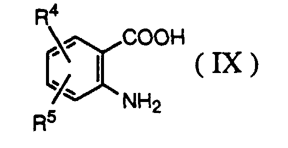 wherein R⁴ and R⁵ have the same meanings as mentioned above with thionyl chloride to react with the amine compound represented by the general formula (V) in the presence of base in an inert solvent.

The compounds represented by the above general formula (IV) using as starting materials in the above production process can be prepared by methods described in the literature or analogous methods thereto or by allowing the carboxylic acid derivative represented by the general formula (II) or a reactive functional derivative thereof such as an acid halide and an activated ester to react with an anthranilic acid derivative represented by the general formula: 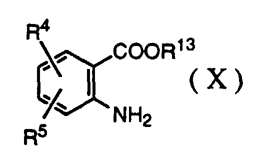 wherein R¹³ represents a hydrogen atom or an alkyl group; and R⁴ and R⁵ have the same meanings as mentioned above in the presence of a base in the presence or absence of a dehydrating agent or a condensing agent in an inert solvent, if desired, converting the ester group into a carboxyl group by hydrolysis, and ring-closing the resulting 2-acylaminobenzoic acid derivative represented by the above general formula (VI) in the presence of a dehydrating agent or a condensing agent such as acetic anhydride or by dehydration on heating.

The compounds represented by the above general formulae (VII), (IX) and (X) used as starting materials in the above production processes are available commercially or can be prepared by methods described in the literature or analogous methods thereto.

Of the 2-acylaminobenzamide derivatives represented by the above general formula (I) of the present invention, compounds having an amino group, a substituted amino group, a hydroxy group or a carboxyl group can be converted into pharmaceutically acceptable salts thereof in the usual way. Examples of such salts include acid addition salts with mineral acids such as hydrochloric acid, hydrobromic acid, hydroiodic acid, sulfuric acid, nitric acid, phosphoric acid and the like, acid addition salts with organic acids such as formic acid, acetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid and the like, salts with inorganic bases such as a sodium salt, a potassium salt and the like, and salts with organic amines such as morpholine, piperidine and the like or amino acids.

In addition, the compounds represented by the above general formula (I) of the present invention also include hydrates thereof and solvates thereof with pharmaceutically acceptable solvents such as ethanol.

Of the compounds represented by the above general formula (I) of the present invention, the compounds having an unsaturated bond exist in two geometrical isomer forms. In the present invention, either the cis (*Z*) isomer or trans (*E*) isomer can be employed, and the trans (*E*) isomer is preferred.

Of the compounds represented by the above general formula (I) of the present invention, in the compounds having an asymmetric carbon atom, two isomer forms of (*R*) configuration and (*S*) configuration exist. Either of the isomers or a mixture thereof can be employed in the present invention.

In the compounds of the present invention, compounds wherein the substituent X represents a vinylene group or a methylene group are preferred, and compounds wherein the substituent X represents a vinylene group are preferred specially.

In the compounds of the present invention, as examples of preferred compounds, (*E*)-2-(3-ethoxycinnamoylamino)benzamide, (*E*)-2-(3-isopropoxycinnamoylamino)benzamide, (*E*)-2-(3,4-dimethoxycinnamoylamino)benzamide, (*E*)-2-(2,3-dimethoxycinnamoylamino)benzamide, (*E*)-2-(3-benzyloxycinnamoylamino)benzamide, (*E*)-2-(4-ethoxycinnamoylamino)benzamide, (*E*)-2-(3-methoxycinnamoylamino)benzamide, (*E*)-2-(2,4-dimethoxycinnamoylamino)benzamide, (*E*)-2-(2,5-dimethoxycinnamoylamino)benzamide, (*E*)-2-(3-ethoxy-4-methoxycinnamoylamino)benzamide, (*E*)-2-(4-ethoxy-3-methoxycinnamoylamino)benzamide, (*E*)-*N*-butyl-2-(3,4-dimethoxycinnamoylamino)benzamide, (*E*)-2-(3,4-dimethoxycinnamoylamino)-*N*-(3-phenylpropyl)benzamide, (*E*)-2-(2-methoxycinnamoylamino)benzamide, (*E*)-2-(2,4,5-trimethoxycinnamoylamino)benzamide, (*E*)-2-(3,4,5-trimethoxycinnamoylamino)benzamide, (*E*)-2-(3,4-methylenedioxycinnamoylamino)benzamide, (*E*)-*N*-[2-(2-hydroxyethoxy)ethyl]-2-(3,4,5-trimethoxycinnamoylamino)benzamide, (*E*)-*N*-(2-hydroxyethyl)-2-(3,4,5-trimethoxycinnamoylamino)benzamide, (*E*)-2-(3,4-dimethoxycinnamoylamino)-*N*-(2-hydroxyethyl)benzamide, (*E*)-*N*- [2-[bis(2-hydroxyethyl)amino]ethyl]-2-(3,4,5-trimethoxycinnamoylamino)benzamide, (*E*)-4,5-dimethoxy-2-(3,4,5-trimethoxycinnamoylamino)benzamide, (*E*)-2-(3,4-dimethoxycinnamoylamino)-*N*-methylbenzamide, (*E*)-*N*-cyclohexylmethyl-2-(3,4-dimethoxycinnamoylamino)benzamide, (*E*)-4,5-dimethoxy-2-(4-ethoxy-3-methoxycinnamoylamino)benzamide, (*E*)-4,5-dimethoxy-2-(3,4-dimethoxycinnamoylamino)benzamide, 2-(3,4-dimethoxyphenylacetylamino)benzamide, 2-(3,4,5-trimethoxyphenylacetylamino)benzamide, 2-(3,5-dimethoxybenzoylamino)benzamide, (*E*)-2-(3,4-dimethoxycinnamoylamino)-*N*-(2-dimethylaminoethyl)benzamide, (*E*)-*N*-(2-dimethylaminoethyl)-2-(3,4,5-trimethoxycinnamoylamino)benzamide and pharmaceutically acceptable salts thereof can be illustrated.

In the compounds of the present invention, as examples of compounds having excellent absorbing property, (*E*)-2-(3,4-dimethoxycinnamoylamino)benzamide, (*E*)-2-(2,5-dimethoxycinnamoylamino)benzamide, (*E*)-2-(3,4,5-trimethoxycinnamoylamino)benzamide, (*E*)-*N*-(2-hydroxyethyl)-2-(3,4,5-trimethoxycinnamoylamino)benzamide, (*E*)-2-(3,4-dimethoxycinnamoylamino)-*N*-(2-hydroxyethyl)benzamide, (*E*)-4,5-dimethoxy-2-(3,4,5-trimethoxycinnamoylamino)benzamide, 2-(3,4-dimethoxyphenylacetylamino)benzamide, 2-(3,4,5-trimethoxyphenylacetylamino)benzamide, (*E*)-2-(3,4-dimethoxycinnamoylamino)-*N*-(2-dimethylaminoethyl)benzamide hydrochloride, (*E*)-*N*-(2-dimethylaminoethyl)-2-(3,4,5-trimethoxycinnamoylamino) benzamide hydrochloride can be illustrated.

In the compounds of the present invention, as examples of specially preferred compounds, (*E*)-2-(3,4,5-trimethoxycinnamoylamino)benzamide, (*E*)-*N*-(2-hydroxyethyl)-2-(3,4,5-trimethoxycinnamoylamino)-benzamide, (*E*)-*N*-(2-dimethylaminoethyl)-2-(3,4,5-trimethoxycinnamoylamino)benzamide hydrochloride can be illustrated.

In the *in vitro* cells proliferation inhibitory activity test using spontaneous hypertensive rat-derived vascular smooth muscle cells in thoracic aorta, the compounds represented by the above general formula (I) of the present invention exhibited a 50% inhibitory activity on vascular smooth muscle cells proliferation at a concentration of about from 1 to 100 µM. On the other hand, in the same test, Tranilast exhibited a 50% inhibitory activity on vascular smooth muscle cells proliferation at a concentration of 231 µM. Thus, the compounds of the present invention have a very potent inhibitory effect on vascular smooth muscle cells proliferation.

In the *in vivo* test using a model of thrombosis-induced neointimal hyperplasia in mouse femoral artery, the compounds of the present invention exhibited an excellent inhibitory effect on neointimal hyperplasia.

As a consequence, the compounds represented by the above general formula (I) of the present invention and pharmaceutically acceptable salts thereof have excellent inhibitory activities on excessive proliferation of vascular intimal cells, and therefore are very useful compounds as agents for the prevention or treatment of diseases caused by excessive proliferation of vascular intimal cells.

As diseases caused by excessive proliferation of vascular intimal cells, for example, restenosis of coronary arteries after PTCA (percutaneous transluminal coronary angioplasty) surgery, restenosis after DCA (directional coronary atherectomy) surgery, restenosis after stent implantation, restenosis after vascular autotransplantation and artificial vascular transplantation, arteriosclerosis and the like can be included.

The compounds represented by the general formula (I) of the present invention have an LD₅₀ value extremely preferable to that of Tranilast and therefore are very safer compounds. For example, in an acute toxicity test using mice, no death was observed by single administration at a dose of 2000mg/kg of 2-(3,4-dimethoxycinnamoylamino)benzamide, and abnormal behavior was also not observed.

When the 2-acylaminobenzamide derivatives represented by the above general formula (I) of the present invention and pharmaceutically acceptable salts thereof are employed in practical treatment, they are administered orally or parenterally in the form of appropriate pharmaceutical compositions such as tablets, powders, granules, capsules, injections and the like. These pharmaceutical compositions can be formulated in accordance with conventional methods using conventional pharmaceutical carriers, excipients and other additives.

Of the above pharmaceutical compositions, in formulating tablets, powders, granules, capsules and the like, conventional excipients, disintegrators, binders, lubricants and the like can be used. Sugars or sugar alcohols such as D-mannitol, lactose and sucrose, starches or starch derivatives such as wheat starch, rice starch, corn starch, potato starch, pregelatinized starch, partly pregelatinized starch, dextrin, cyclodextrin, pullulan, hydroxypropylstarch and the like, celluloses or cellulose derivatives such as crystalline cellulose, crystalline cellulose·carmellose sodium, methylcellulose, hydroxypropylmethylcellulose and the like, sodium alginate, acacia, agar, macrogol, aluminium stearate, aluminium monostearate and the like can be used as excipients, and calcium hydrogenphosphate, anhydrous calcium hydrogenphosphate, magnesium aluminometasilicate, synthetic aluminum silicate, synthetic hydrotalcite, aluminum hydroxide, magnesium hydroxide, calcium phosphate, dried aluminum hydroxide gel, precipitated calcium carbonate, light anhydrous silicic acid and the like can be used as mineral excipients. The application of these materials is not limited to excipients, and these materials can be used as disintegrators or binders.

Carmellose calcium, carmellose, low substituted hydroxypropylcellulose, sodium carboxymethylstarch, croscarmellose sodium, tragacanth, starches or starch derivatives such as wheat starch, rice starch, corn starch, potato starch, pregelatinized starch, partly pregelatinized starch, dextrin, pullulan, hydroxypropylstarch and the like can be used as disintegrators. The application of these materials is not limited to disintegrators, and these materials can be used as excipients.

Hydroxyethylcellulose, hydroxypropylcellulose, polyvinyl alcohol, polyvinylpyrrolidone, starches or starch derivatives such as wheat starch, rice starch, corn starch, potato starch, pregelatinized starch, partly pregelatinized starch, dextrin, pullulan, hydroxypropylstarch and the like can be used as binders.

Calcium stearate, magnesium stearate, stearic acid, talc, cetanol, polyoxy 40 stearate, leucine, hydrogenated oil, sodium lauryl sulfate, paraffin, polyoxyethyleneglycol fatty acid ester, fatty acid ester and the like can be used as lubricants. The application of these materials is not limited to lubricants, and these materials can be used as excipients.

In formulating tablets, tablets can be coated with film using lactose, saccharose, gelatin, hydroxypropylcellulose, hydroxypropylmethylcellulose, polyvinylacetal diethylaminoacetate, metaacrylic acid copolymer, hydroxypropylmethylcellulose phthalate or the like.

In formulating injections, distilled water, isotonic sodium chloride solution, alcohol, glycerin, poly alcohol, vegetable oil and the like can be used as diluents. These preparations may further contain buffers, isotonicities, antiseptics, moistening agents, emulsifiers, dispersing agents, stabilizing agents, dissolving aids and the like.

The dosage is appropriately decided depending on the sex, age, body weight, degree of symptoms and the like of each patient to be treated, which is approximately within the range of from 0.1 to 1,000 mg per day per adult human in the case of oral administration and approximately within the range of from 0.01 to 100 mg per day per adult human in the case of parenteral administration, and the daily dose is divided into one to several doses per day.

### Examples

The present invention is further illustrated in more detail by way of the following Reference Examples and Examples. The present invention is not limited thereto.

### Reference Example 1

### Isopropyl (E)-3-isopropoxycinnamate

To a suspension of (*E*)-3-hydroxycinnamic acid (2 g) and potassium carbonate (3.7 g) in *N*,*N*-dimethylformamide (20 ml) was added isopropyl iodide (2.68 ml), and the mixture was stirred at 80 °C for 3 days. To the reaction mixture was added water, and the mixture was extracted with ethyl acetate. The extract was washed with an aqueous saturated sodium hydrogen carbonate solution, dried over anhydrous magnesium sulfate and concentrated under reduced pressure. Separation and purification of the residue by column chromatography on silica gel (eluent: methylene chloride) gave isopropyl (*E*)-3 isopropoxycinnamate (1.20 g).
¹H-NMR (CDCl₃, 400MHz) δ ppm:
   1.31 (d, J=6.3Hz, 6H), 1.34 (d, J=6.1Hz, 6H), 4.5-4.65 (m, 1H), 5.05-5.2 (m, 1H), 6.39 (d, J=16.0Hz, 1H), 6.8-7.3 (m, 4H), 7.62 (d, J=16.0Hz, 1H)

### Reference Example 2

In the similar manner to that in Reference Example 1, the following compounds were prepared.

### Ethyl (E)-3,5-dimethoxy-4-ethoxycinnamate

¹H-NMR (CDCl₃, 400MHz) δ ppm:
   1.25-1.5 (m, 6H), 3.87 (s, 6H), 4.09 (q, J=7.1Hz, 2H), 4.27 (q, J=7.1Hz, 2H), 6.35 (d, J=15.9Hz, 1H), 6.75 (s, 2H), 7.61 (d, J=15.9Hz, 1H)

### 2-Hydroxyethyl (E)-3,5-dimethoxy-4-(2-hydroxyethoxy)cinnamate

¹H-NMR (CDCl₃, 400MHz) δ ppm:
   3.7-4.45 (m, 14H), 6.41 (d, J=15.9Hz, 1H), 6.77 (s, 2H), 7.65 (d, J=15.9Hz, 1H)

### Reference Example 3

### (E)-3-Isopropoxycinnamic acid

To a solution of isopropyl (*E*)-3-isopropoxycinnamate (1.1 g) in ethanol (10 ml) was added 2 N aqueous sodium hydroxide solution (5 ml), and the mixture was stirred for 15 hours at room temperature. The reaction mixture was acidified with 1 N hydrochloric acid and extracted with ethyl acetate. The extract was dried over anhydrous magnesium sulfate and concentrated under reduced pressure to give (*E*)-3-isopropoxycinnamic acid (914 mg).
¹H-NMR (CDCl₃, 400MHz) δ ppm:
   1.36 (d, J=6.0Hz, 6H), 4.5-4.65 (m, 1H), 6.43 (d, J=16.0Hz, 1H), 6.9-7.4 (m, 4H), 7.75 (d, J=16.0Hz, 1H)

### Reference Example 4

In the similar manner to that in Reference Example 3, the following compounds were prepared.

### (E)-3,5-Dimethoxy-4-ethoxycinnamic acid

¹H-NMR (CDCl₃, 400MHz) δ ppm:
   1.37 (t, J=7.1Hz, 3H), 3.89 (s, 6H), 4.11 (q, J=7.1Hz, 2H), 6.36 (d, J=15.9Hz, 1H), 6.78 (s, 2H), 7.71 (d, J=15.9Hz, 1H)

### (E)-3,5-Dimethoxy-4-(2-hydroxyethoxy)cinnamic acid

¹H-NMR (CDCl₃, 400MHz) δ ppm:
   3.7-3.8 (m, 2H), 3.91 (s, 6H), 4.1-4.25 (m, 2H), 6.37 (d, J=15.9Hz, 1H), 6.79 (s, 2H), 7.70 (d, J=15.9Hz, 1H)

### 4-Ethoxy-3-methoxyphenylacetic acid

¹H-NMR (CDCl₃, 400MHz) δ ppm:
   1.45 (t, J=7.0Hz, 3H), 3.59 (s, 2H), 3.87 (s, 3H), 4.08 (q, J=7.0Hz, 2H), 6.7-6.9 (m, 3H)

### Reference Example 5

### tert-Butyl (E)-2-ethoxycarbonylcinnamate

To *N*,*N*-dimethylformamide (10 ml) were added ethyl o-bromobenzoate (1.0 g), sodium iodide (720 mg) and nickel (II) bromide (190 mg), and the mixture was stirred at 140 °C for 4 hours. To the reaction mixture were added *tert*-butyl acrylate (0.7 ml), palladium (0) bis(dibenzylideneacetone) (23 mg), tri-o-tolylphosphine (530 mg) and triethylamine (0.67 ml), and the mixture was stirred at 140 °C for 3 hours. The reaction mixture was diluted with methylene chloride, and the precipitated complex was removed by filtration. The filtrate was washed with a dilute acetic acid, and the solvent was removed under reduced pressure. To the residue was added diethyl ether, and the mixture was washed with distilled water 3 times and dried over anhydrous magnesium sulfate. The solvent was removed under reduced pressure, and the residue was separated and purified by column chromatography on silica gel (eluent: methylene chloride) to give *tert*-butyl

### (E)-2-ethoxycarbonylcinnamate (676 mg).

¹H-NMR (CDCl₃, 400MHz) δ ppm:
   1.41 (t, J=7.1Hz, 3H), 1.54 (s, 9H), 4.40 (q, J=7.1Hz, 2H), 6.23 (d, J=15.9Hz, 1H), 7.35-7.65 (m, 3H), 7.94 (dd, J=7.8, 1.4Hz, 1H), 8.31 (d, J=15.9Hz, 1H)

### Reference Example 6

### (E)-2-Ethoxycarbonylcinnamic acid

*tert*-Butyl (*E*)-2-ethoxycarbonylcinnamate (538 mg) was added to trifluoroacetic acid (5 ml), and the mixture was stirred at 40 °C for 30 minutes. The solvent was removed under reduced pressure, and a small amount of diethyl ether was added to the residue. The mixture was sonicated and collected by filtration to give (*E*)-2-ethoxycarbonylcinnamic acid (339 mg).
¹H-NMR (DMSO-*d*_{*6*}, 400MHz) δ ppm:
   1.34 (t, J=7.1Hz, 3H), 4.34 (q, J=7.1Hz, 2H), 6.43 (d, J=15.9Hz, 1H), 7.45-7.95 (m, 4H), 8.23 (d, J=15.9Hz, 1H), 12.4-12.65 (br, 1H)

### Reference Example 7

### tert-Butyl α-cyano-3,4-dimethoxycinnamate

To a solution of 3,4-dimethoxybenzaldehyde (1.7 g) and *tert*-butyl cyanoacetate (2.0 g) in ethanol (30 ml) was added a catalytic amount of piperidine, and the mixture was heated under reflux for 30 minutes. Ice-water was added to the reaction mixture, and the resulting precipitates were collected by filtration and washed with water to give *tert*-butyl α-cyano-3,4-dimethoxycinnamate (2.23 g).
¹H-NMR (DMSO-*d*_{*6*}, 400MHz) δ ppm:
   1.52 (s, 9H), 3.80 (s, 3H), 3.87 (s, 3H), 7.17 (d, J=8.5Hz, 1H), 7.6-7.8 (m, 2H), 8.21 (s, 1H)

### Reference Example 8

### α-Cyano-3,4-dimethoxycinnamic acid

Trifluoroacetic acid (10 ml) was added to *tert*-butyl α-cyano-3,4-dimethoxycinnamate (1.01 g) under ice-cooling, and the mixture was stirred for 30 minutes at room temperature. The reaction mixture was concentrated under reduced pressure, and water was added to the residue. The resulting insoluble material was collected by filtration to give α-cyano-3,4-dimethoxycinnamic acid (810 mg).
¹H-NMR (DMSO-*d*_{*6*}, 400MHz) δ ppm:
   3.80 (s, 3H), 3.87 (s, 3H), 7.18 (d, J=8.6Hz, 1H), 7.70 (dd, J=8.6, 2.1Hz, 1H), 7.76 (d, J=2.1Hz, 1H), 8.25 (s, 1H)

### Reference Example 9

### β-Cyano-3,4-dimethoxycinnamic acid

To a solution of 3,4-dimethoxyphenylacetonitrile (5.0 g) and glyoxylic acid (3.9 g) in methanol (60 ml) was added potassium carbonate (9.7 g), and the mixture was heated under reflux for 2 days. The reaction mixture was cooled to room temperature, and the precipitates were collected by filtration, washed with methylene chloride and diethyl ether, and dissolved in water (50 ml). The aqueous solution was washed with diethyl ether and acidified with an aqueous citric acid solution. The resulting precipitates were collected by filtration and washed with water to give β-cyano-3,4-dimethoxycinnamic acid (540 mg).
¹H-NMR (CDCl₃, 270MHz) δ ppm:
   3.94 (s, 3H), 3.95 (s, 3H), 6.6-7.8 (m, 4H)

### Reference Example 10

### (E)-3,4-Ethylenedioxycinnamic acid

To a solution of 3,4-ethylenedioxybenzaldehyde (2.0 g) and malonic acid (1.5 g) in pyridine (20 ml) was added piperidine (24 µl), and the mixture was heated under reflux for 20 hours. Malonic acid (7.5 g) was added to the reaction mixture again, and the mixture was heated under reflux for 20 hours. The reaction mixture was concentrated under reduced pressure, and the residue was dissolved in 2 N aqueous sodium hydroxide solution. The aqueous solution was washed with methylene chloride and acidified with concentrated hydrochloric acid. The resulting precipitates were collected by filtration and washed with water and hexane to give (*E*)-3,4-ethylenedioxycinnamic acid (1.48 g).
¹H-NMR (DMSO-*d*_{*6*}, 400MHz) δ ppm:
   4.2-4.4 (m, 4H , 6.37 (d, J=16.0Hz, 1H), 6.89 (d, J=8.4 Hz, 1H), 7.18 (dd, J=8.4, 2.0Hz, 1H), 7.24 (d, J=2.0Hz, 1H), 7.48 (d, J=16.0Hz, 1H), 12.0-12.6 (br, 1H)

### Reference Example 11

### (E)-2-(2-Carboxycinnamoylamino)benzoic acid

To a solution of (*E*)-2-(2-ethoxycarbonylcinnamoyl)aminobenzamide (510.8 mg) in acetic acid (2 ml) was added concentrated hydrochloric acid (2 ml), and the mixture was heated at 100 °C for 5 minutes. After the solvent was removed under reduced pressure, ethanol (2 ml) and 1 N aqueous sodium hydroxide solution (2 ml) were added to the residue, and the mixture was heated at 100 °C for 4 hours. The reaction mixture was acidified with 1 N hydrochloric acid, and the resulting precipitates were collected by filtration and washed with diethyl ether to give (*E*)-2-(2-carboxycinnamoylamino)benzoic acid (144 mg).
¹H-NMR (DMSO-*d*_{*6*}, 400MHz) δ ppm:
   6.76 (d, J=15.6Hz, 1H), 7.1-8.05 (m, 7H), 8.34 (d, J=15.6Hz, 1H), 8.59 (d, J=7.5Hz, 1H), 11.40 (brs, 1H), 13.0-14.0 (br, 2H)

### Reference Example 12

### (E)-4-Acetoxy-3,5-dimethoxycinnamic acid

To a solution of (*E*)-3,5-dimethoxy-4-hydroxycinnamic acid (365 mg) in acetic anhydride (5 ml) was added sodium hydroxide (65 mg), and the mixture was heated at 100 °C for 20 minutes. The reaction mixture was concentrated under reduced pressure, and the residue was acidified with 2 N hydrochloric acid, and the mixture was extracted with ethyl acetate. The extract was dried over anhydrous magnesium sulfate and concentrated under reduced pressure to give (*E*)-4-acetoxy-3,5-dimethoxycinnamic acid (334 mg).
¹H-NMR (DMSO-*d*_{*6*}, 400MHz) δ ppm:
   2.26 (s, 3H), 3.81 (s, 6H), 6.64 (d, J=16.0Hz, 1H), 7.12 (s, 2H), 7.57 (d, J=16.0Hz, 1H), 12.3-12.5 (br, 1H)

### Reference Example 13

### (E)-4-(2-Acetoxyethoxy)-3,5-dimethoxycinnamic acid

To a solution of (*E*)-3,5-dimethoxy-4-(2-hydroxyethoxy)cinnamic acid (292 mg) in pyridine (6 ml) was added acetic anhydride (0.31 ml), and the mixture was stirred for 13 hours at room temperature. The solvent was removed under reduced pressure, and water was added to the residue, and the mixture was extracted with ethyl acetate. The extract was dried over anhydrous magnesium sulfate and concentrated under reduced pressure. Separation and purification of the residue by column chromatography on silica gel (eluent: methylene chloride/diethyl ether = 1/1) gave (*E*)-4-(2-acetoxyethoxy)-3,5-dimethoxycinnamic acid (60 mg).
¹H-NMR (CDCl₃, 400MHz) δ ppm:
   2.09 (s, 3H), 3.88 (s, 6H), 4.2-4.4 (m, 4H), 6.36 (d, J=15.9Hz, 1H), 6.78 (s, 2H), 7.70 (d, J=15.9Hz, 1H)

### Reference Example 14

### 2-Amino-N-(3-phenylpropyl)benzamide

To a solution of isatoic anhydride (500 mg) in *N*,*N*-dimethylformamide (3 ml) were added 4-dimethylaminopyridine (37 mg) and 3-phenylpropylamine (436 mg), and the mixture was stirred for 1 day at room temperature. To the reaction mixture was added water, and the mixture was extracted with ethyl acetate. The extract was dried over anhydrous magnesium sulfate and concentrated under reduced pressure to give 2-amino-*N*-(3-phenylpropyl)benzamide (760 mg).
¹H-NMR (CDCl₃, 400MHz) δ ppm:
   1.9-2.0 (m, 2H), 2.65-2.8 (m, 2H), 3.4-3.55 (m, 2H), 5.48 (brs, 2H), 5.98 (brs, 1H), 6.55-6.95 (m, 2H), 7.1-7.4 (m, 7H)

### Reference Example 15

### 2-Amino-N-[2-(2-hydroxyethoxy)ethyl]benzamide

A solution of isatoic anhydride (1.0 g) and 2-(2-aminoethoxy)ethanol (645 mg) in ethanol (5 ml) was heated under reflux for 10 minutes. The solvent was removed under reduced pressure, and the residue was separated and purified by column chromatography on silica gel (eluent: chloroform/methanol = 10/1) to give 2-amino-*N*-[2-(2-hydroxyethoxy)ethyl]benzamide (1.32 g).
¹H-NMR (CDCl₃, 400MHz) δ ppm:
   2.05-2.45 (br, 1H), 3.55-3.85 (m, 8H), 5.25-5.75 (br, 2H), 6.50-6.75 (m, 3H), 7.15-7.25 (m, 1H), 7.35 (dd, J=7.9, 1.5Hz, 1H)

### Reference Example 16

In the similar manner to that in Reference Example 15, the following compounds were prepared.

### 2-Amino-N-(2-hydroxyethyl)benzamide

¹H-NMR (CDCl₃, 400MHz) δ ppm:
   2.66 (t, J=5.1Hz, 1H), 3.50-3.65 (m, 2H), 3.75-3.9 (m, 2H), 5.50 (brs, 2H), 6.40-6.75 (m, 3H), 7.15-7.30 (m, 1H), 7.34 (dd, J=7.9, 1.5Hz, 1H)

### 2-Amino-N-(4-hydroxybutyl)benzamide

¹H-NMR (CDCl₃, 400MHz) δ ppm:
   1.40-2.00 (m, 4H), 3.40-3.50 (m, 2H), 3.72 (t, J=6.0Hz, 2H), 5.30-5.70 (br, 2H), 6.34 (brs, 1H), 6.55-6.75 (m, 2H), 7.10-7.40 (m, 2H)

### 2-Amino-N-(2-dimethylaminoethyl)benzamide

¹H-NMR (CDCl₃, 400MHz) δ ppm:
   2.26 (s, 6H), 2.50 (t, J=6.0Hz, 2H), 3.40-3.55 (m, 2H), 5.54 (brs, 2H), 6.60-6.80 (m, 3H), 7.15-7.25 (m, 1H), 7.35 (dd, J=7.8, 1.4Hz, 1H)

### 2-Amino-N-[2-[bis(2-hydroxyethyl)amino]ethyl]benzamide

¹H-NMR (CDCl₃, 400MHz) δ ppm:
   2.61 (t, J=5.0Hz, 4H), 2.69 (t, J=5.5Hz, 2H), 3.4-3.5 (m, 2H), 3.57 (t, J=5.0Hz, 4H), 5.41 (brs, 2H), 6.6-7.35 (m, 4H), 7.40 (dd, J=7.8, 1.3Hz, 1H)

### Reference Example 17

### (E)-2-(4-Dimethylaminocinnamoylamino)benzoic acid

To a solution of *N*-carboxymethylcarbonylanthranilic acid (5 g) and 4-dimethylaminobenzaldehyde (3.43 g) in toluene (50 ml) was added piperidine (2.22 ml), and the mixture was heated under reflux for 18 hours in a vessel equipped with Dean-Stark trap. Toluene was removed under reduced pressure, and the residue was separated and purified by column chromatography on silica gel (eluent: ethyl acetate). The resulting crystals were washed with diethyl ether to give (*E*)-2-(4-dimethylaminocinnamoylamino)benzoic acid (1.14 g).
¹H-NMR (DMSO-*d*_{*6*}, 400MHz) δ ppm:
   2.98 (s, 6H), 6.54 (d, J=15.5Hz, 1H), 6.73 (d, J=8.9Hz, 2H), 7.1-7.7 (m, 5H), 8.00 (dd, J=7.9, 1.6Hz, 1H), 8.63 (d, J=8.4Hz, 1H), 11.25 (s, 1H), 13.3-13.9 (br, 1H)

### Reference Example 18

### (E)-2-(4-Dimethylaminostyryl)-3,1-benzoxazin-4-one

(*E*)-2-(4-Dimethylaminocinnamoylamino)benzoic acid (500 mg) was dissolved in acetic anhydride (10 ml), and the solution was stirred for 1 hour at room temperature. The solvent was removed under reduced pressure to give (*E*)-2-(4-dimethylaminostyryl)-3,1-benzoxazin-4-one (480 mg).
¹H-NMR (CDCl₃, 400MHz) δ ppm:
   3.04 (s, 6H), 6.56 (d, J=15.9Hz, 1H), 6.65-7.85 (m, 8H), 8.19 (dd, J=7.9, 1.4Hz, 1H)

### Reference Example 19

### (E)-6,7-Dimethoxy-2-(3,4-dimethoxystyryl)-3,1-benzoxazin-4-one

To a solution of 4,5-dimethoxyanthranilic acid (435 mg) in pyridine (10 ml) was added (*E*)-3,4-dimethoxycinnamoyl chloride (500 mg), and the mixture was stirred for 2 hours at room temperature. Acetic anhydride (642 mg) was added to the reaction mixture, and the mixture was stirred for 13 hours at room temperature. The reaction mixture was poured into water, and the mixture was stirred. The resulting precipitates were collected by filtration to give (*E*)-6,7-dimethoxy-2-(3,4-dimethoxystyryl)-3,1-benzoxazin-4-one (635 mg).
¹H-NMR (DMSO-*d*_{*6*}, 400MHz) δ ppm:
   3.81 (s, 3H), 3.83 (s, 3H), 3.89 (s, 3H), 3.94 (s, 3H), 6.85 (d, J=16.1Hz, 1H), 7.00 (d, J=8.4Hz, 1H), 7.09 (s, 1H), 7.25-7.5 (m, 3H), 7.67 (d, J=16.1Hz, 1H)

### Reference Example 20

### (E)-6,7-Dimethoxy-2-(3,4,5-trimethoxystyryl)-3,1-benzoxazin-4-one

To a solution of 4,5-dimethoxyanthranilic acid (423 mg) in pyridine (5 ml) was added (*E*)-3,4,5-trimethoxycinnamoyl chloride (500 mg), and the mixture was stirred for 1 hour at room temperature. Acetic anhydride (510 mg) was added to the reaction mixture, and the mixture was stirred for 13 hours at room temperature. The reaction mixture was poured into water, and the mixture was stirred. The resulting precipitates were collected by filtration, and separated and purified by column chromatography on silica gel (eluent: methylene chloride/diethyl ether = 10/1) to give (*E*)-6,7-dimethoxy-2-(3,4,5-trimethoxystyryl)-3,1-benzoxazin-4-one (562 mg).
¹H-NMR (CDCl₃, 400MHz) δ ppm:
   3.90 (s, 3H), 3.91 (s, 3H), 4.00 (s, 3H), 4.02 (s, 3H), 6.68 (d, J=16.0Hz, 1H), 6.82 (s, 2H), 7.02 (s, 2H), 7.55 (s, 2H), 7.75(d, J=16.0Hz, 1H)

### Reference Example 21

### (E)-2-(2-Carboxystyryl)-3,1-benzoxazin-4-one

To a solution of (*E*)-2-(2-carboxycinnamoylamino)benzoic acid (70 mg) in pyridine (1 ml) was added acetic anhydride (42.5 µl), and the mixture was stirred for 3 days at room temperature. The reaction mixture was acidified with 1 N hydrochloric acid, and the resulting precipitates were collected by filtration and washed with 1 N hydrochloric acid and water to give (*E*)-2-(2-carboxystyryl)-3,1-benzoxazin-4-one (32.6 mg).
¹H-NMR (DMSO-*d*_{*6*}, 400MHz) δ ppm:
   6.92 (d, J=16.1Hz, 1H), 7.5-8.2 (m, 8H), 8.53 (d, J=16.1Hz, 1H), 13.33 (brs, 1H)

### Reference Example 22

In the similar manner to that in Reference Examples 18-21, the following compounds were prepared.

### (E)-6,7-Dimethoxy-2-(4-ethoxy-3-methoxystyryl)-3,1-benzoxazin-4-one

¹H-NMR (DMSO-*d*_{*6*}, 400MHz) δ ppm:
   1.34 (t, J=6.9Hz, 3H), 3.83 (s, 3H), 3.89 (s, 3H), 3.94 (s, 3H), 4.06 (q, J=6.9Hz, 2H), 6.83 (d, J=16.1Hz, 1H), 6.98 (d, J=8.4Hz, 1H), 7.08 (s, 1H), 7.20-7.5 (m, 3H), 7.66 (d, J=16.1Hz, 1H)

### (E)-2-(3,4-Dimethoxystyryl)-6-methoxy-3,1-benzoxazin-4-one

¹H-NMR (CDCl₃, 400MHz) δ ppm:
   3.85-4.0 (m, 9H), 6.66 (d, J=16.0Hz, 1H), 6.91 (d, J=8.3Hz, 1H), 7.1-7.2 (m, 2H), 7.38 (dd, J=8.9, 3.0Hz, 1H), 7.53 (d, J=8.9Hz, 1H), 7.61 (d, J=3.0Hz, 1H), 7.76 (d, J=16.0Hz, 1H)

### (E)-6-Acetoxy-2-(3,4,5-trimethoxystyryl)-3,1-benzoxazin-4-one

¹H-NMR (DMSO-*d*_{*6*}, 400MHz) δ ppm:
   2.32 (s, 3H), 3.71 (s, 3H), 3.85 (s, 6H), 7.05 (d, J=16.1Hz, 1H), 7.19 (s, 2H), 7.65-7.9 (m, 4H)

### (E)-8-Acetoxy-2-(3,4,5-trimethoxystyryl)-3,1-benzoxazin-4-one

¹H-NMR (CDCl₃, 400MHz) δ ppm:
   2.47 (s, 3H), 3.91 (s, 3H), 3.93 (s, 6H), 6.67 (d, J=16.0Hz, 1H), 6.83 (s, 2H), 7.45-7.6 (m, 2H), 7.76 (d, J=16.0Hz, 1H), 8.12 (dd, J=7.6, 1.7Hz, 1H)

### (E)- 2-(3,4-Dimethoxystyryl)-6-methoxy-3,1-benzoxazin-4-one

¹H-NMR (CDCl₃, 400MHz) δ ppm:
   3.92 (s, 3H), 3.936 (s, 3H), 3.938 (s, 3H), 6.66 (d, J=16.0Hz, 1H), 6.91 (d J=8.3Hz, 1H), 7.1-7.45 (m, 3H), 7.53 (d, J=8.9Hz, 1H), 7.61 (d, J=2.9Hz, 1H), 7.76 (d, J=16.0Hz, 1H)

### Reference Example 23

### Methyl (E)-2-(3,4-dimethoxycinnamoylamino)-5-hydroxybenzoate

(*E*)-3,4-Dimethoxycinnamoyl chloride (147 mg) and methyl 5-hydroxyanthranilate (100 mg) were added to pyridine (2 ml), and the mixture was stirred at 80 °C for 5 hours. The reaction mixture was poured into water, and the mixture was stirred. The resulting precipitates were collected by filtration and washed with 1 N hydrochloric acid and water to give methyl (*E*)-2-(3,4-dimethoxycinnamoylamino)-5-hydroxybenzoate (100 mg).
¹H-NMR (DMSO-*d*_{*6*}, 400MHz) δ ppm:
   3.80 (s, 3H), 3.83 (s, 3H), 3.85 (s, 3H), 6.78 (d J=15.5Hz, 1H), 6.95-7.4 (m, 5H), 7.51 (d, J=15.5Hz, 1H), 8.16 (d, J=9.0Hz, 1H), 9.68 (brs, 1H), 10.42 (s, 1H)

### Example 1

### (E)-2-(3-Ethoxycinnamoylamino)benzamide (Compound 1)

To a suspension of (*E*)-3-ethoxycinnamic acid (448 mg) in toluene (2.5 ml) were added thionyl chloride (826 mg) and a catalytic amount of *N*,*N*-dimethylformamide, and the mixture was heated at 80 °C for 3 hours. The reaction mixture was concentrated under reduced pressure, and 2-aminobenzamide (314 mg) and pyridine (8 ml) were added to the residue, and the mixture was heated under reflux at 130 °C for 3 hours. The reaction mixture was poured into water, and the mixture was stirred. The resulting precipitates were collected by filtration and washed with 1 N hydrochloric acid, an aqueous saturated sodium hydrogen carbonate solution, water and diethyl ether successively to give (*E*)-2-(3-ethoxycinnamoylamino)benzamide (458 mg).
¹H-NMR (CDCl₃, 400MHz) δ ppm:
   1.4-1.5 (m, 3H), 4.08 (q, J=7.0Hz, 2H), 6.48 (brs, 1H), 6.59 (d, J=15.5Hz, 1H), 6.8-7.8 (m, 9H), 8.76 (d, J=8.4Hz, 1H), 11.86 (brs, 1H)

### Example 2

### (E)-2-(3-Isopropoxycinnamoylamino)benzamide (Compound 2)

To a suspension of (*E*)-3-isopropoxycinnamic acid (500 mg) in toluene (5 ml) were added thionyl chloride (3 ml) and a drop of *N*,*N*-dimethylformamide, and the mixture was stirred at 80 °C for 30 minutes. The reaction mixture was concentrated under reduced pressure, and the residue was dissolved in tetrahydrofuran (10 ml). To the solution was added 2-aminobenzamide (693 mg) under ice-cooling, and the mixture was stirred for 5 minutes at room temperature. To the reaction mixture was added water, and the mixture was extracted with ethyl acetate. The extract was washed with 1 N hydrochloric acid, an aqueous saturated sodium hydrogen carbonate solution and brine successively, dried over anhydrous magnesium sulfate and concentrated under reduced pressure to give (*E*)-2-(3-isopropoxycinnamoylamino)benzamide (738 mg).
¹H-NMR (DMSO-*d*_{*6*}, 400MHz) δ ppm:
   1.28 (d, J=6.0Hz, 6H), 4.65-4.8 (m, 1H), 6.83 (d, J=15.6Hz, 1H), 6.9-7.9 (m, 9H), 8.30 (brs, 1H), 8.57 (dd, J=8.4, 1.0Hz, 1H), 11.85 (s, 1H)

### Example 3

### (E)-2-(2-Ethoxycarbonylcinnamoylamino)benzamide (Compound 3)

To a solution of (*E*)-2-ethoxycarbonylcinnamic acid (295 mg) in toluene (2 ml) were added thionyl chloride (195 µl) and a drop of *N*,*N*-dimethylformamide, and the mixture was stirred at 50 °C for 1 hour. The solvent was removed under reduced pressure, and the residue was dissolved in tetrahydrofuran (1 ml). To the solution was added dropwise a solution of 2-aminobenzamide (456 mg) in tetrahydrofuran (1 ml) under ice-cooling, and the mixture was stirred for 5 minutes at room temperature. The precipitated salt was removed by filtration, and the filtrate was concentrated under reduced pressure. The residue was washed with 1 N hydrochloric acid, 1 N aqueous sodium hydroxide solution, distilled water and diethyl ether successively to give (*E*)-2-(2-ethoxycarbonylcinnamoylamino)benzamide (418 mg).
¹H-NMR (CDCl₃, 400MHz) δ ppm:
   1.41 (t, J=7.1Hz, 3H), 4.42 (q, J=7.1Hz, 2H), 5.3-6.4 (br, 2H), 6.46 (d, J=15.6Hz, 1H), 7.0-7.7 (m, 6H), 7.95 (dd, J=7.9, 1.2Hz, 1H), 8.44 (d, J=15.6Hz, 1H), 8.82 (dd, J=8.7, 1.1Hz, 1H), 11.52 (s, 1H)

### Example 4

In the similar manner to that in Examples 1-3, the following compounds were prepared.

### (E)-2-(3,4-Dimethoxycinnamoylamino)benzamide (Compound 4)

¹H-NMR (DMSO-*d*_{*6*}, 400MHz) δ ppm:
   3.80 (s, 3H), 3.84 (s, 3H), 6.71 (d, J=15.7Hz, 1H), 6.95-7.9 (m, 8H), 8.28 (brs, 1H), 8.59 (dd, J=8.4, 0.9Hz, 1H), 11.80 (s, 1H)

### (E)-2-(2,3-Dimethoxycinnamoylamino)benzamide (Compound 5)

¹H-NMR (DMSO-*d*_{*6*}, 400MHz) δ ppm:
   3.78 (s, 3H), 3.83 (s, 3H), 6.77 (d, J=15.9Hz, 1H), 7.0-7.9 (m, 8H), 8.31 (brs, 1H), 8.57 (dd, J=8.4, 1.0Hz, 1H), 11.98 (s, 1H)

### (E)-2-(3-Benzyloxycinnamoylamino)benzamide (Compound 6)

¹H-NMR (DMSO-*d*_{*6*}, 400MHz) δ ppm:
   5.17 (s, 2H), 6.85 (d, J=15.6Hz, 1H), 7.0-7.9 (m, 14H), 8.31 (brs, 1H), 8.5-8.65 (m, 1H), 11.88 (s, 1H)

### (E)-2-(3-Butoxycinnamoylamino)benzamide (Compound 7)

¹H-NMR (DMSO-*d*_{*6*}, 400MHz) δ ppm:
   0.95 (t, J=7.4Hz, 3H), 1.4-1.8 (m, 4H), 4.03 (t, J=6.5Hz, 2H), 6.85 (d, J=15.7Hz, 1H), 6.9-7.9 (m, 9H), 8.30 (brs, 1H), 8.57 (dd, J=8.3, 0.9Hz, 1H), 11.85 (s, 1H)

### (E)-2-(4-Methoxycinnamoylamino)benzamide (Compound 8)

¹H-NMR (DMSO-*d*_{*6*}, 400MHz) δ ppm:
   3.80 (s, 3H), 6.64 (d, J=15.6Hz, 1H), 6.9-7.9 (m, 9H), 8.27 (brs, 1H), 8.57 (dd, J=8.3, 0.9Hz, 1H), 11.84 (s, 1H)

### (E)-2-(4-Ethoxycinnamoylamino)benzamide (Compound 9)

¹H-NMR (DMSO-*d*_{*6*}, 400MHz) δ ppm:
   1.34 (t, J=6.9Hz, 3H), 4.08 (q, J=6.9Hz, 2H), 6.63 (d, J=15.6Hz, 1H), 6.9-7.85 (m, 9H), 8.27 (brs, 1H), 8.5-8.7 (m, 1H), 11.84 (s, 1H)

### (E)-2-(3-Cyclohexylmethoxycinnamoylamino)benzamide (Compound 10)

¹H-NMR (DMSO-*d*_{*6*}, 400MHz) δ ppm:
   0.8-2.0 (m, 11H), 3.84 (d, J=6.1Hz, 2H), 6.5-8.1 (m, 10H), 8.32 (brs, 1H), 8.57 (d, J=8.2Hz, 1H), 11.85 (s, 1H)

### (E)-2-(3-Methoxycinnamoylamino)benzamide (Compound 11)

¹H-NMR (DMSO-*d*_{*6*}, 400MHz) δ ppm:
   3.81 (s, 3H), 6.84 (d, J=15.7Hz, 1H), 6.9-7.9 (m, 9H), 8.30 (brs, 1H), 8.57 (d, J=8.2Hz, 1H), 11.87 (s, 1H)

### (E)-2-(2,4-Dimethoxycinnamoylamino)benzamide (Compound 12)

¹H-NMR (CDCl₃, 400MHz) δ ppm:
   3.84 (s, 3H), 3.88 (s, 3H), 5.2-6.2 (br, 2H), 6.46 (d, J=2.4Hz, 1H), 6.51 (dd, J=8.4, 2.3Hz, 1H), 6.61 (d, J=15.6Hz, 1H), 7.0-7.6 (m, 4H), 7.95 (d, J=15.7Hz, 1H), 8.8-8.9 (m, 1H), 11.29 (brs, 1H)

### (E)-2-(2,5-Dimethoxycinnamoylamino)benzamide (Compound 13)

¹H-NMR (DMSO-*d*_{*6*}, 400MHz) δ ppm:
   3.77 (s, 3H), 3.82 (s, 3H), 6.82 (d, J=15.7Hz, 1H), 6.9-8.6 (m, 10H), 11.86 (s, 1H)

### (E)-2-(3-Nitrocinnamoylamino)benzamide (Compound 14)

¹H-NMR (DMSO-*d*_{*6*}, 400MHz) δ ppm:
   7.0-8.7 (m, 12H) 11.89 (s, 1H)

### (E)-2-(4-Methylcinnamoylamino)benzamide (Compound 15)

¹H-NMR (DMSO-*d*_{*6*}, 400MHz) δ ppm:
   2.33 (s, 3H), 6.73 (d, J=15.6Hz, 1H), 7.1-7.9 (m, 9H), 8.29 (brs, 1H), 8.57 (dd, J=8.3, 0.9Hz, 1H), 11.89 (s, 1H)

### (E)-2-(3-Ethoxy-4-methoxycinnamoylamino)benzamide (Compound 16)

¹H-NMR (DMSO-*d*_{*6*}, 400MHz) δ ppm:
   1.35 (t, J=6.9Hz, 3H), 3.79 (s, 3H), 4.09 (q, J=6.9Hz, 2H), 6.70 (d, J=15.5Hz, 1H), 6.9-7.9 (m, 8H), 8.29 (brs, 1H), 8.58 (dd, J=8.4, 1.0Hz, 1H), 11.78 (s, 1H)

### (E)-2-(4-Ethoxy-3-methoxycinnamoylamino)benzamide (Compound 17)

¹H-NMR (DMSO-*d*_{*6*}, 400MHz) δ ppm:
   1.33 (t, J=6.9Hz, 3H), 3.83 (s, 3H), 4.05 (q, J=6.9Hz, 2H), 6.71 (d, J=15.5Hz, 1H), 6.9-8.4 (m, 9H), 8.59 (dd, J=8.4, 1.0Hz, 1H), 11.80 (s, 1H)

### (E)-2-(4-Cyanocinnamoylamino)benzamide (Compound 18)

¹H-NMR (DMSO-*d*_{*6*}, 400MHz) δ ppm:
   7.03 (d, J=15.6Hz, 1H), 7.1-8.4 (m, 10H), 8.57 (dd, J=8.3, 0.9Hz, 1H), 11.95 (s, 1H)

### (E)-N-Butyl-2-(3,4-dimethoxycinnamoylanino)benzamide (Compound 19)

¹H-NMR (CDCl₃, 400MHz) δ ppm:
   0.98 (t, J=7.3Hz, 3H), 1.3-1.8 (m, 4H), 3.46 (td, J=7.1, 5.7Hz, 2H), 3.92 (s, 3H), 3.96 (s, 3H), 6.28 (brs, 1H), 6.48 (d, J=15.5Hz, 1H), 6.87 (d, J=8.2Hz, 1H), 7.0-7.6 (m, 5H), 7.68 (d, J=15.6Hz, 1H), 8.75 (dd, J=8.4, 0.9Hz, 1H), 11.30 (s, 1H)

### (E)-2-(3,4-Dimethoxycinnanoylamino)-N-(3-phenylpropyl)benzamide (Compound 20)

¹H-NMR (CDCl₃, 400MHz) δ ppm:
   1.95-2.05 (m, 2H), 2.76 (t, J=7.4Hz, 2H), 3.45-3.55 (m, 2H), 3.92 (s, 3H), 3.95 (s, 3H), 6.28 (brs, 1H), 6.47 (d, J=15.5Hz, 1H), 6.8-7.55 (m, 11H), 7.67 (d, J=15.5Hz, 1H), 8.65-8.8 (m, 1H), 11.29 (s, 1H)

### (E)-2-(2-Ethoxycinnamoylamino)benzamide (Compound 21)

¹H-NMR (CDCl₃, 400MHz) δ ppm:
   1.50 (t, J=6.9Hz, 3H), 4.12 (q, J=6.9Hz, 2H), 6.26 (brs, 1H), 6.73 (d, J=15.7Hz, 1H), 6.8-7.8 (m, 8H), 8.03 (d, J=15.7Hz, 1H), 8.79 (dd, J=8.4, 0.8Hz, 1H), 11.71 (s, 1H)

### (E)-2-(3-Chlorocinnamoylamino)benzamide (Compound 22)

¹H-NMR (DMSO-*d*_{*6*}, 400MHz) δ ppm:
   6.95 (d, J=15.6Hz, 1H), 7.0-8.6 (m, 11H), 11.88 (s, 1H)

### (E)-2-(3-Fluorocinnamoylamino)benzamide (Compound 23)

¹H-NMR (DMSO-*d*_{*6*}, 400MHz) δ ppm:
   6.93 (d, J=15.6Hz, 1H), 7.1-7.95 (m, 9H), 8.32 (brs, 1H), 8.58 (dd, J=8.3, 0.9Hz, 1H), 11.91 (s, 1H)

### (E)-2-(2-Methoxycinnamoylamino)benzamide (Compound 24)

¹H-NMR (DMSO-*d*_{*6*}, 400MHz) δ ppm:
   3.88 (s, 3H), 6.76 (d, J=15.7Hz, 1H), 6.9-7.9 (m, 9H) 8.30 (brs, 1H), 8.58 (dd, J=8.3, 0.9Hz, 1H), 11.92 (s, 1H)

### (E)-2-(2,4,5-Trimethoxycinnamoylamino)benzamide (Compound 25)

¹H-NMR (DMSO-*d*_{*6*}, 400MHz) δ ppm:
   3.77 (s, 3H), 3.84 (s, 3H), 3.87 (s, 3H), 6.68 (d, J=15.6Hz, 1H), 6.72 (s, 1H), 7.0-7.9 (m, 6H), 8.28 (brs, 1H), 8.58 (d, J=8.3Hz, 1H), 11.75 (s, 1H)

### (E)-2-(3,4,5-Trimethoxycinnamoylamino)benzamide (Compound 26)

¹H-NMR (DMSO-*d*_{*6*}, 400MHz) δ ppm:
   3.69 (s, 3H), 3.84 (s, 6H), 6.83 (d, J=15.5Hz, 1H), 7.07 (s, 2H), 7.1-8.7 (m, 7H), 11.80 (s, 1H)

### (E)-2-Cinnamoylaminobenzamide (Compound 27)

¹H-NMR (DMSO-*d*_{*6*}, 400MHz) δ ppm:
   6.80 (d, J=15.7Hz, 1H), 7.1-7.9 (m, 10H), 8.28 (brs, 1H), 8.57 (dd, J=8.4, 0.9Hz, 1H), 11.91 (s, 1H)

### (E)-2-(4-Chlorocinnamoylamino)benzamide (Compound 28)

¹H-NMR (DMSO-*d*_{*6*}, 400MHz) δ ppm:
   6.85 (d, J=15.6Hz, 1H), 7.0-7.9 (m, 9H), 8.28 (brs, 1H), 8.57 (d, J=7.8Hz, 1H), 11.90 (s, 1H)

### (E)-2-(4-Hydroxy-3-methoxycinnamoylamino)benzamide (Compound 29)

¹H-NMR (CDCl₃, 400MHz) δ ppm:
   3.98 (s, 3H), 6.09 (brs, 1H), 6.58 (d, J=15.8Hz, 1H), 6.9-8.2 (m, 8H)

### (E)-2-(3,4-Methylenedioxycinnamoylamino)benzamide (Compound 30)

¹H-NMR (DMSO-*d*_{*6*}, 400MHz) δ ppm:
   6.20 (s, 2H), 6.80 (d, J=15.5Hz, 1H), 7.07 (d, J=8.0Hz, 1H), 7.2-8.0 (m, 7H), 8.39 (brs, 1H), 8.70 (dd, J=8.4, 1.0Hz, 1H), 11.94 (s, 1H)

### (E)-2-(2,3,4-Trimethoxycinnamoylamino)benzamide (Compound 31)

¹H-NMR (DMSO-*d*_{*6*}, 400MHz) δ ppm:
   3.77 (s, 3H), 3.84 (s, 3H), 3.85 (s, 3H), 6.68 (d, J=15.8Hz, 1H), 6.89 (d, J=8.8Hz, 1H), 7.05-8.4 (m, 7H), 8.59 (d, J=8.3Hz, 1H), 11.93 (s, 1H)

### (E)-2-(3,5-Dimethoxycinnamoylamino)benzamide (Compound 32)

¹H-NMR (DMSO-*d*_{*6*}, 400MHz) δ ppm:
   3.80 (s, 6H), 6.45-7.9 (m, 9H), 8.30 (brs, 1H), 8.57 (d, J=7.6Hz, 1H), 11.83 (s, 1H)

### (E)-2-(4-Acetylcinnamoylamino)benzamide (Compound 33)

¹H-NMR (DMSO-*d*_{*6*}, 400MHz) δ ppm:
   2.60 (s, 3H), 6.96 (d, J=15.7Hz, 1H), 7.1-7.85 (m, 5H), 7.87 (d, J=8.4Hz, 2H), 7.98 (d, J=8.4Hz, 2H), 8.28 (brs, 1H), 8.57 (dd, J=8.4, 1.0Hz, 1H), 11.94 (s, 1H)

### (E)-2-(4-Methylsulfonylcinnamoylamino)benzamide (Compound 34)

¹H-NMR (DMSO-*d*_{*6*}, 400MHz) δ ppm:
   3.24 (s, 3H), 7.01 (d, J=15.7Hz, 1H), 7.1-7.85 (m, 5H), 7.95 (d, J=8.6Hz, 2H), 7.99 (d, J=8.6Hz, 2H), 8.28 (brs, 1H), 8.56 (dd, J=8.4, 1.0Hz, 1H), 11.94 (s, 1H)

### (E)-2-(3,5-Dimethoxy-4-ethoxycinnamoylamino)benzamide (Compound 35)

¹H-NMR (CDCl₃, 400MHz) δ ppm:
   1.37 (t, J=7.1Hz, 3H), 3.90 (s, 6H), 4.10 (q, J=7.1Hz, 2H), 5.45-6.45 (br, 2H), 6.51 (d, J=15.5Hz, 1H), 6.80 (s, 2H), 7.05-7.6 (m, 3H), 7.65 (d, J=15.5Hz, 1H), 8.82 (dd, J=8.8, 1.1Hz, 1H), 11.44 (s, 1H)

### 2-(3-Phenylpropioloylamino)benzamide (Compound 36)

¹H-NMR (DMSO-*d*_{*6*}, 400MHz) δ ppm:
   7.1-8.5 (m, 11H), 12.39 (brs, 1H)

### (E)-2-(2,4,6-Trimethoxycinnamoylamino)benzamide (Compound 37)

¹H-NMR (DMSO-*d*_{*6*}, 400MHz) δ ppm:
   3.84 (s, 3H), 3.88 (s, 6H), 6.31 (s, 2H), 6.78 (d, J=15.8Hz, 1H), 7.05-7.85 (m, 4H), 7.91 (d, J=15.8Hz, 1H), 8.27 (brs, 1H), 8.60 (dd, J=8.4, 1.0Hz, 1H), 11.86 (s, 1H)

### (E)-2-(3,4-Ethylenedioxycinnamoylamino)benzamide (Compound 38)

¹H-NMR (DMSO-*d*_{*6*}, 400MHz) δ ppm:
   4.2-4.4 (m, 4H), 6.65 (d, J=15.6Hz, 1H), 6.89 (d, J=8.4Hz, 1H), 7.1-7.9 (m, 7H), 8.29 (brs, 1H), 8.57 (d, J=8.4Hz, 1H), 11.81 (s, 1H)

### Example 5

### (E)-2-(3,4-Dimethoxycinnamoylamino)-N-(2-dimethylaminoethyl)benzamide (Compound 39)

To a solution of 2-amino-*N*-(2-dimethylaminoethyl)benzamide (500 mg) in tetrahydrofuran (10 ml) were added triethylamine (370 µl) and (E)-3,4-dimethoxycinnamoyl chloride (600 mg), and the mixture was stirred for 15 hours at room temperature. To the reaction mixture was added water, and the mixture was extracted with ethyl acetate. The extract was dried over anhydrous magnesium sulfate and concentrated under reduced pressure. Separation and purification of the residue by column chromatography on aminopropyl silica gel (eluent: ethyl acetate) gave (*E*)- 2-(3,4-dimethoxycinnamoylamino)-*N*-(2-dimethylaminoethyl)benzamide (416 mg).
¹H-NMR (CDCl₃, 400MHz) δ ppm:
   2.28 (s, 6H), 2.55 (t, J=5.9Hz, 2H), 3.45-3.55 (m, 2H), 3.92 (s, 3H), 3.96 (s, 3H), 6.49 (d, J=15.5Hz, 1H), 6.87 (d, J=8.3Hz, 1H), 7.00-7.20 (m, 4H), 7.45-7.55 (m, 2H), 7.69 (d, J=15.5Hz, 1H), 8.78 (d, J=8.0Hz, 1H), 11.48 (s, 1H)

### Example 6

In the similar manner to that in Example 5, the following compounds were prepared.

### (E)-N-(2-Dimethylaminoethyl)-2-(4-ethoxy-3-methoxycinnamoylamino)benzamide (Compound 40)

¹H-NMR (CDCl₃, 400MHz) δ ppm:
   1.49 (t, J=7.0Hz, 3H), 2.29 (s, 6H), 2.55 (t, J=5.9Hz, 2H), 3.45-3.6 (m, 2H), 3.95 (s, 3H), 4.15 (q, J=7.0Hz, 2H), 6.49 (d, J=15.5Hz, 1H), 6.87 (d, J=8.4Hz, 1H), 6.95-7.6 (m, 6H), 7.68 (d, J=15.5Hz, 1H), 8.79 (d, J=8.7Hz, 1H), 11.47 (s, 1H)

### (E)-N-(2-Dimethylaminoethyl)-2-(3,4,5-trimethoxycinnamoylamino)benzamide (Compound 41)

¹H-NMR (CDCl₃, 400MHz) δ ppm:
   2.29 (s, 6H), 2.56 (t, J=5.9Hz, 2H), 3.45-3.60 (m, 2H), 3.88 (s, 3H), 3.92 (s, 6H), 6.53 (d, J=15.5Hz, 1H), 6.80 (s, 2H), 7.05-7.6 (m, 4H), 7.65 (d, J=15.5Hz, 1H), 8.78(d, J=8.3Hz, 1H), 11.51 (s, 1H)

### (E)-2-(3,4-Dimethoxycinnamoylamino)-N-[2-(2-hydroxyethoxy)ethyl]benzamide (Compound 42)

¹H-NMR (CDCl₃, 400MHz) δ ppm:
   3.05-3.15 (m, 1H), 3.60-3.85 (m, 8H), 3.93 (s, 3H), 3.97 (s, 3H), 6.43 (d, J=15.5Hz, 1H), 6.88 (d, J=8.3Hz, 1H), 6.95-7.05 (m, 1H), 7.12 (d, J=1.8Hz, 1H), 7.17 (dd, J=8.3, 1.8Hz, 1H), 7.35-7.50 (m, 3H), 7.69 (d, J=15.5Hz, 1H), 8.56 (d, J=8.3Hz, 1H), 11.15 (s, 1H)

### (E)-N-[2-(2-Hydroxyethoxy)ethyl]-2-(3,4,5-trimethoxycinnamoylamino)benzamide (Compound 43)

¹H-NMR (CDCl₃, 400MHz) δ ppm:
   2.90-3.00 (m, 1H), 3.60-3.85 (m, 8H), 3.89 (s, 3H), 3.93 (s, 6H), 6.48 (d, J=15.5Hz, 1H), 6.82 (s, 2H), 6.95-7.05 (m, 1H), 7.25-7.55 (m, 3H), 7.66 (d, J=15.5Hz, 1H), 8.55-8.65 (m, 1H), 11.21 (s, 1H)

### (E)-2-(4-Ethoxy-3-methoxycinnamoylamino)-N-[2-(2-hydroxyethoxy)ethyl]benzamide (Compound 44)

¹H-NMR (CDCl₃, 400MHz) δ ppm:
   1.49 (t, J=7.0Hz, 3H), 2.85-3.00 (br, 1H), 3.60-3.85 (m, 8H), 3.95 (s, 3H), 4.15 (q, J=7.0Hz, 2H), 6.43 (d, J=15.5Hz, 1H), 6.87 (d, J=8.2Hz, 1H), 6.95-7.5 (m, 6H), 7.68 (d, J=15.5Hz, 1H), 8.59 (d, J=8.3Hz, 1H), 11.16 (s, 1H)

### (E)-N-(2-Hydroxyethyl)-2-(3,4,5-trimethoxycinnnamoylamino)benzamide (Compound 45)

¹H-NMR (CDCl₃, 400MHz) δ ppm:
   2.37 (t, J=5.0Hz, 1H), 3.55-3.70 (m, 2H), 3.80-4.00 (m, 11H), 6.50 (d, J=15.5Hz, 1H), 6.80 (s, 2H), 6.85-6.95 (m, 1H), 7.00-7.15 (m, 1H), 7.45-7.55 (m, 2H), 7.65 (d, J=15.5Hz, 1H), 8.65-8.75 (m, 1H), 11.26 (s, 1H)

### (E)-2-(3,4-Dimethoxycinnamoylamino)-N-(2-hydroxyethyl)benzamide (Compound 46)

¹H-NMR (CDCl₃, 400MHz) δ ppm:
   2.40-2.65 (br, 1H), 3.60-3.95 (m, 7H), 3.96 (s, 3H), 6.45 (d, J=15.5Hz, 1H), 6.80-7.20 (m, 5H), 7.40-7.55 (m, 2H), 7.68 (d, J=15.5Hz, 1H), 8.6-8.75 (m, 1H), 11.20 (s, 1H)

### (E)-2-(3,4-Dimethoxycinnamoylamino)-N-(4-hydroxybutyl)benzamide (Compound 47)

¹H-NMR (DMSO-*d*_{*6*}, 400MHz) δ ppm:
   1.40-1.70 (m, 4H), 3.20-3.50 (m, 4H), 3.80 (s, 3H), 3.84 (s, 3H), 4.40 (t, J=5.1Hz, 1H), 6.75 (d, J=15.5Hz, 1H), 6.99 (d, J=8.4Hz, 1H), 7.10-7.60 (m, 5H), 7.75 (dd, J=7.9, 1.4Hz, 1H), 8.55 (dd, J=8.4, 1.0Hz, 1H), 8.70-8.80 (m, 1H), 11.49 (s, 1H)

### (E)-N-(4-Hydroxybutyl)-2-(3,4,5-trimethoxycinnamoylamino)benzamide (Compound 48)

¹H-NMR (DMSO-*d*_{*6*}, 400MHz) δ ppm:
   1.40-1.70 (m, 4H), 3.20-3.50 (m, 4H), 3.70 (s, 3H), 3.85 (s, 6H), 4.41 (t, J=5.1Hz, 1H), 6.85 (d, J=15.5Hz, 1H), 7.08(s, 2H), 7.10-7.60 (m, 3H), 7.75 (dd, J=7.9, 1.4Hz, 1H), 8.55 (dd, J=8.4, 1.0Hz, 1H), 8.70-8.85 (m, 1H), 11.52 (s, 1H)

### (E)-2-(3-Ethoxy-4-methoxycinnamoylamino)-N-(2-hydroxyethyl)benzamide (Compound 49)

¹H-NMR (CDCl₃, 400MHz) δ ppm:
   1.52 (t, J=7.0Hz, 3H), 2.44 (t, J=5.1Hz, 1H), 3.55-3.70 (m, 2H), 3.80-4.00 (m, 5H), 4.17 (q, J=7.0Hz, 2H), 6.44 (d, J=15.5Hz, 1H), 6.80-7.20 (m, 5H), 7.40-7.55 (m, 2H), 7.67 (d, J=15.5Hz, 1H), 8.65-8.75 (m, 1H), 11.20 (s, 1H)

### (E)-N-[2-[Bis(2-hydroxyethyl)amino]ethyl]-2-(3,4-dimethoxycinnamoylamino)benzamide (Compound 50)

¹H-NMR (CDCl₃, 400MHz) δ ppm:
   2.45-3.65 (m, 12H), 3.92 (s, 3H), 3.96 (s, 3H), 6.46 (d, J=15.5Hz, 1H), 6.87 (d, J=8.3Hz, 1H), 6.95-7.45 (m, 4H), 7.54 (dd, J=7.8, 1.2Hz, 1H), 7.67 (d, J=15.5Hz, 1H), 8.05 (t, J=5.3Hz, 1H), 8.47 (d, J=8.3Hz, 1H), 11.11 (s, 1H)

### (E)-N-[2-[Bis(2-hydroxyethyl)amino]ethyl]-2-(3,4,5-trimethoxycinnamoylamino)benzamide (Compound 51)

¹H-NMR (CDCl₃, 400MHz) δ ppm:
   2.56 (t, J=4.6Hz, 4H), 2.69 (t, J=5.1Hz, 2H), 3.45-3.65 (m, 6H), 3.89 (s, 3H), 3.92 (s, 6H), 6.51 (d, J=15.5Hz, 1H), 6.81 (s, 2H), 6.95-7.5 (m, 2H), 7.55 (dd, J=7.9, 1.3Hz, 1H), 7.65 (d, J=15.5Hz, 1H), 8.04 (t, J=5.3Hz, 1H), 8.50 (dd, J=8.3, 0.8Hz, 1H), 11.18 (s, 1H)

### Example 7

### 2-(α-Cyano-3,4-dimethoxycinnamoylamino)benzamide (Compound 52)

To a solution of α-cyano-3,4-dimethoxycinnamic acid (200 mg) in toluene (3 ml) were added oxalyl chloride (0.44 ml) and a catalytic amount of *N*,*N*-dimethylformamide under ice-cooling, and the mixture was stirred for 1 hour under ice-cooling. The reaction mixture was concentrated under reduced pressure at room temperature, and 2-aminobenzamide (120 mg) and pyridine (3 ml) were added to the residue, and the mixture was stirred for 5 hours at room temperature. The reaction mixture was concentrated under reduced pressure, and water was added to the residue. The resulting precipitates were collected by filtration and washed with an aqueous citric acid solution, water and diethyl ether successively to give 2-(α-cyano-3,4-dimethoxycinnamoylamino)benzamide (170 mg).
¹H-NMR (DMSO-*d*_{*6*}, 400MHz) δ ppm:
   3.83 (s, 3H), 3.88 (s, 3H), 7.1-8.0 (m, 7H), 8.33 (s, 1H), 8.39 (brs, 1H), 8.57 (dd, J=8.4, 0.9Hz, 1H), 12.86 (s, 1H)

### Example 8

In the similar manner to that in Example 7, the following compound was prepared.

### 2-(β-Cyano-3,4-dimethoxycinnamoylamino)benzamide (Compound 53)

¹H-NMR (CDCl₃, 400MHz) δ ppm:
   3.95 (s, 3H), 3.97 (s, 3H), 5.4-5.8 (br, 1H), 6.1-6.5 (br, 1H), 6.9-7.7 (m, 7H), 8.90 (dd, J=8.9, 1.1Hz, 1H), 11.76 (s, 1H)

### Example 9

### (E)-2-(3,4-Dimethoxycinnamoylamino)-N,N-dimethylbenzamide (Compound 54)

To a mixture of (*E*)-2-(3,4-dimethoxystyryl)-3,1-benzoxazin-4-one (100 mg) and dimethylamine hydrochloride (80 mg) were added pyridine (1 ml) and triethylamine (0.13 ml), and the mixture was heated at 115 °C for 18 hours. The reaction mixture was acidified with 2 N hydrochloric acid and extracted with ethyl acetate. The extract was washed with an aqueous saturated sodium hydrogen carbonate solution and brine, dried over anhydrous magnesium sulfate and concentrated under reduced pressure to give (*E*)-2-(3,4-dimethoxycinnamoylamino)-*N*,*N*-dimethylbenzamide (95 mg).
¹H-NMR (CDCl₃, 400MHz) δ ppm:
   3.0-3.25 (m, 6H), 3.92 (s, 3H), 3.95 (s, 3H), 6.44 (d, J=15.5Hz, 1H), 6.8-7.8 (m, 7H), 8.39 (d, J=8.3Hz, 1H), 9.34 (s, 1H)

### Example 10

### (E)-2-(4-Dimethylaminocinnamoylamino)benzamide (Compound 55)

To a solution of (*E*)-2-(4-dimethylaminostyryl)-3,1-benzoxazin-4-one in methanol (5 ml) and methylene chloride (5 ml) was added 28% aqueous ammonium hydroxide solution (2 ml), and the mixture was heated at 50 °C for 10 minutes. The solvent was removed under reduced pressure, and the residue was washed with methylene chloride to give (*E*)-2-(4-dimethylaminocinnamoylamino)benzamide (24 mg).
¹H-NMR (DMSO-*d*_{*6*}, 400MHz) δ ppm:
   2.98 (s, 6H), 6.46 (d, J=15.5Hz, 1H), 6.72 (d, J=8.8Hz, 2H), 7.05-7.6 (m, 5H), 7.70 (brs, 1H), 7.80 (d, J=6.9Hz, 1H), 8.27 (brs, 1H), 8.59 (d, J=8.1Hz, 1H), 11.79 (s, 1H)

### Example 11

### (E)-4,5-Dimethoxy-2-(3,4-dimethoxycinnamoylamino)-N-(2-hydroxyethyl)benzamide (Compound 56)

To a solution of (*E*)-6,7-dimethoxy-2-(3,4-dimethoxystyryl)-3,1-benzoxazin-4-one (100 mg) in *N*,*N*-dimethylformamide (2 ml) was added 2-aminoethanol (50 mg), and the mixture was stirred for 24 hours at room temperature. The reaction mixture was concentrated under reduced pressure, and the residue was crystallized with methylene chloride and diethyl ether. The resulting crystals were collected by filtration and washed with diethyl ether to give (*E*)-4,5-dimethoxy-2-(3,4-dimethoxycinnamoylamino)-*N*-(2-hydroxyethyl)benzamide (87 mg).
¹H-NMR (DMSO-*d*_{*6*}, 400MHz) δ ppm:
   3.3-3.45 (m, 2H), 3.5-3.65 (m, 2H), 3.79 (s, 3H), 3.80 (s, 3H), 3.81 (s, 3H), 3.84 (s, 3H), 4.80 (t, J=5.6Hz, 1H), 6.71 (d, J=15.5Hz, 1H), 6.98 (d, J=8.5Hz, 1H), 7.15-7.45 (m, 3H), 7.51 (d, J=15.5Hz, 1H), 8.40 (s, 1H), 8.67 (t, J=5.5Hz, 1H), 11.96 (s, 1H)

### Example 12

### (E)-4,5-Dimethoxy-2-(3,4,5-trimethoxycinnamoylamino)benzamide (Compound 57)

(*E*)-6,7-Dimethoxy-2-(3,4,5-trimethoxystyryl)-3,1-benzoxazin-4-one (562 mg) was added to 28% aqueous ammonium hydroxide solution (50 ml), and the mixture was stirred for 7 hours at room temperature. The resulting precipitates were collected by filtration and washed with water, a mixed solution of methylene chloride/methanol and methylene chloride successively to give (*E*)-4,5-dimethoxy-2-(3,4,5-trimethoxycinnamoylamino)benzamide (302 mg).
¹H-NMR (DMSO-*d*_{*6*}, 400MHz) δ ppm:
   3.70 (s, 3H), 3.75-3.95 (m, 12H), 6.78 (d, J=15.6Hz, 1H), 7.04 (s, 2H), 7.39 (s, 1H), 7.45-7.65 (m, 2H), 8.21 (brs, 1H), 8.43 (s, 1H), 12.24 (s, 1H)

### Example 13

In the similar manner to that in Examples 9-12, the following compounds were prepared.

### (E)-2-(3,4-Dimethoxycinnamoylamino)-N-methylbenzamide (Compound 58)

¹H-NMR (CDCl₃, 400MHz) δ ppm:
   3.03 (d, J=4.9Hz, 3H), 3.93 (s, 3H), 3.96 (s, 3H), 6.38 (brs, 1H), 6.49 (d, J=15.5Hz, 1H), 6.8-7.6 (m, 6H), 7.69 (d, J=15.5Hz, 1H), 8.74 (d, J=8.3Hz, 1H), 11.30 (s, 1H)

### (E)-N-Cyclohexylmethyl-2-(3,4-dimethoxycinnamoylamino)benzamide (Compound 59)

¹H-NMR (DMSO-*d*_{*6*}, 400MHz) δ ppm:
   0.8-1.8 (m, 11H), 3.1-3.2 (m, 2H), 3.80 (s, 3H), 3.84 (s, 3H), 6.74 (d, J=15.5Hz, 1H), 6.9-7.8 (m, 7H), 8.52 (d, J=8.4Hz, 1H), 8.7-8.8 (m, 1H), 11.44 (s, 1H)

### (E)-2-(3,4-Dimethoxycinnamoylamino)-N-hydroxybenzamide (Compound 60)

¹H-NMR (CDCl₃, 400MHz) δ ppm:
   3.93 (s, 3H), 3.94 (s, 3H), 6.68 (d, J=16.0Hz, 1H), 6.85-7.85 (m, 7H), 8.22 (dd, J=7.9, 1.5Hz, 1H)

### (E)-4,5-Dimethoxy-2-(4-ethoxy-3-methoxycinnamoylamino)-N-(2-hydroxyethyl)benzamide (Compound 61)

¹H-NMR (DMSO-*d*_{*6*}, 400MHz) δ ppm:
   1.33 (t, J=7.0Hz, 3H), 3.2-3.6 (m, 4H), 3.80 (s, 3H), 3.81 (s, 3H), 3.84 (s, 3H), 4.05 (q, J=7.0Hz, 2H), 4.7-4.9 (m, 1H), 6.70 (d, J=15.5Hz, 1H), 6.96 (d, J=8.3Hz, 1H), 7.1-7.4 (m, 3H), 7.51 (d, J=15.5Hz, 1H), 8.40 (s, 1H), 8.6-8.75 (m, 1H), 11.95 (s, 1H)

### (E)-2-(3,4-Dimethoxycinnamoylamino)-N-(2-hydroxyethyl)-5-methoxybenzamide (Compound 62)

¹H-NMR (DMSO-*d*_{*6*}, 400MHz) δ ppm:
   3.2-3.6 (m, 4H), 3.79 (s, 3H), 3.80 (s, 3H), 3.83 (s, 3H), 4.80 (t, J=5.7Hz, 1H), 6.73 (d, J=15.5Hz, 1H), 6.98 (d, J=8.4Hz, 1H), 7.05-7.45 (m, 4H), 7.50 (d, J=15.5Hz, 1H), 8.35-8.8 (m, 2H), 11.09 (s, 1H)

### (E)-5-Hydroxy-2-(3,4,5-trimethoxycinnamoylamino)benzamide (Compound 63)

¹H-NMR (DMSO-*d*_{*6*}, 400MHz) δ ppm:
   3.69 (s, 3H), 3.84 (s, 6H), 6.79 (d, J=15.5Hz, 1H), 6.85-7.2 (m, 4H), 7.48 (d, J=15.5Hz, 1H), 7.60 (brs, 1H), 8.15 (brs, 1H), 8.29 (d, J=8.9Hz, 1H), 9.53 (brs, 1H), 11.14 (s, 1H)

### (E)-3-Hydroxy-2-(3,4,5-trimethoxycinnamoylamino)benzamide (Compound 64)

¹H-NMR (DMSO-*d*_{*6*}, 400MHz) δ ppm:
   3.70 (s, 3H), 3.84 (s, 6H), 6.9-7.6 (m, 8H), 7.90 (brs, 1H), 9.80-9.95 (br, 1H), 10.50-10.65 (br, 1H)

### (E)-4,5-Dimethoxy-2-(4-ethoxy-3-methoxycinnamoylamino)benzamide (Compound 65)

¹H-NMR (DMSO-*d*_{*6*}, 400MHz) δ ppm:
   1.34 (t, J=6.9Hz, 3H), 3.80 (s, 3H), 3.81 (s, 3H), 3.83 (s, 3H), 4.05 (q, J=6.9Hz, 2H), 6.67 (d, J=15.5Hz, 1H), 6.97 (d, J=8.4Hz, 1H), 7.1-7.65 (m, 5H), 8.22 (brs, 1H), 8.44 (s, 1H), 12.23 (s, 1H)

### (E)-4,5-Dimethoxy-2-(3,4-dimethoxycinnamoylamino)benzamide (Compound 66)

¹H-NMR (DMSO-*d*_{*6*}, 400MHz) δ ppm:
   3.65-4.05 (m, 12H), 6.68 (d, J=15.6Hz, 1H), 6.99 (d, J=8.4Hz, 1H), 7.15-7.7 (m, 5H), 8.15-8.25 (br, 1H), 8.43 (s, 1H), 12.23 (s, 1H)

### (E)-2-(3,4-Dimethoxycinnamoylamino)-5-methoxybenzamide (Compound 67)

¹H-NMR (CD₃OD, 400MHz) δ ppm:
   3.89 (s, 3H), 3.96 (s, 3H), 3.99 (s, 3H), 6.56 (d, J=15.6Hz, 1H), 6.97 (d, J=8.5Hz, 1H), 7.05-7.4 (m, 4H), 7.64 (d, J=15.6Hz, 1H), 8.54 (d, J=9.1Hz, 1H)

### Example 14

### (E)-2-(2-Carboxycinnamoylamino)benzamide (Compound 68)

To 28% aqueous ammonium hydroxide solution (1 ml) was added (*E*)-2-(2-carboxystyryl)-3,1-benzoxazin-4-one (20 mg) under ice-cooling, and the mixture was stirred for 10 minutes. The reaction mixture was acidified with 1 N hydrochloric acid, and the resulting precipitates were collected by filtration and washed with water to give (*E*)-2-(2-carboxycinnamoylamino)benzamide (21 mg).
¹H-NMR (DMSO-*d*_{*6*}, 400MHz) δ ppm:
   6.69 (d, J=15.6Hz, 1H), 7.45-7.95 (m, 8H), 8.25-8.40 (m, 2H), 8.59 (d, J=8.4Hz, 1H), 11.95 (s, 1H), 13.24 (brs, 1H)

### Example 15

### (E)-2-(3,5-Dimethoxy-4-hydroxycinnamoylamino)benzamide (Compound 69)

To a solution of (*E*)-4-acetoxy-3,5-dimethoxycinnamic acid (70 mg) in toluene (1 ml) were added thionyl chloride (38 µl) and a drop of *N*,*N*-dimethylformamide, and the mixture was heated at 80 °C for 30 minutes. The reaction mixture was concentrated under reduced pressure, and pyridine (1 ml) and 2-aminobenzamide (43 mg) were added to the residue, and the mixture was heated at 115 °C for 10 minutes. The solvent was removed under reduced pressure, and 2 N hydrochloric acid was added to the residue, and the mixture was extracted with ethyl acetate. The extract was dried over anhydrous magnesium sulfate, and the solvent was removed under reduced pressure. The residue was separated and purified by column chromatography on silica gel (eluent: ethyl acetate) to give (*E*)-2-(4-acetoxy-3,5-dimethoxycinnamoylamino)benzamide. To the obtained compound were added methanol (1 ml) and potassium carbonate (36 mg), and the mixture was stirred for 5 minutes at room temperature. The solvent was removed under reduced pressure, and 2 N hydrochloric acid was added to the residue, and the mixture was extracted with ethyl acetate. The extract was dried over anhydrous magnesium sulfate and concentrated under reduced pressure. Separation and purification of the residue by preparative thin layer chromatography (developing solvent: ethyl acetate) gave (*E*)-2-(3,5-dimethoxy-4-hydroxycinnamoylamino)benzamide (7 mg).
¹H-NMR (DMSO-*d*_{*6*}, 400MHz) δ ppm:
   3.84 (s, 6H), 6.71 (d, J=15.3Hz, 1H), 7.04 (s, 2H), 7.1-7.85 (m, 5H), 8.32 (brs, 1H), 8.61 (d, J=8.3Hz, 1H), 8.90 (brs, 1H), 11.78 (s, 1H)

### Example 16

### 2-[3,5-Dimethoxy-4-(2-hydroxyethoxy)cinnamoylamino]benzamide (Compound 70)

To a solution of (*E*)-4-(2-acetoxyethoxy)-3,5-dimethoxycinnamic acid (57 mg) in toluene (1 ml) were added thionyl chloride (40 µl) and a catalytic amount of *N*,*N*-dimethylformamide, and the mixture was heated at 80 °C for 1 hour. The solvent was removed under reduced pressure, and pyridine (1 ml) and 2-aminobenzamide (25 mg) were added to the residue, and the mixture was heated under reflux at 130 °C for 2 hours. The solvent was removed under reduced pressure, and water was added to the residue, and the mixture was extracted with ethyl acetate. The extract was washed with 1 N hydrochloric acid, an aqueous saturated sodium hydrogen carbonate solution, water and brine successively and dried over anhydrous magnesium sulfate. The solvent was removed under reduced pressure, and the residue was dissolved in methanol (0.5 ml). To the solution was added potassium carbonate (17 mg), and the mixture was stirred for 1 hour at room temperature. To the reaction mixture was added 1 N hydrochloric acid (0.5 ml), and the solvent was removed under reduced pressure. The residue was washed with water and diethyl ether, and separated and purified by preparative thin layer chromatography (developing solvent: methylene chloride/diethyl ether/ methanol = 10/10/1) to give 2-[3,5-dimethoxy-4-(2-hydroxyethoxy)cinnamoylamino]benzamide (5 mg).
¹H-NMR (CDCl₃+CD₃OD, 400MHz) δ ppm:
   3.7-3.85 (m, 2H), 3.94 (s, 6H), 4.1-4.25 (m, 2H), 6.57 (d, J=15.6Hz, 1H), 6.86 (s, 2H), 7.1-7.6 (m, 2H), 7.61 (d, J=15.6Hz, 1H), 7.70 (dd, J=7.9, 1.4Hz, 1H), 8.69 (dd, J=8.4, 0.9Hz, 1H)

### Example 17

### 2-[3-(3,4-Dimethoxyphenyl)propionylamino]benzamide (Compound 71)

Methanol (10 ml) was added to a mixture of (*E*)-2-(3,4-dimethoxycinnamoylamino)benzamide (300 mg) and 10 % palladium on carbon (30 mg), and the mixture was allowed to react for 18 hours in a stream of hydrogen under atmospheric pressure at room temperature. The insoluble material was removed by filtration, and the filtrate was concentrated under reduced pressure to give 2-[3-(3,4-dimethoxyphenyl)propionylamino]benzamide (289 mg).
¹H-NMR (CDCl₃, 400MHz) δ ppm:
   2.70 (t, J=7.3Hz, 2H), 3.01 (t, J=7.3Hz, 2H), 3.83 (s, 3H), 3.84 (s, 3H), 5.4-6.3 (br, 2H), 6.7-7.55 (m, 6H), 8.64 (dd, J=8.8, 1.1Hz, 1H), 11.11 (s, 1H)

### Example 18

In the similar manner to that in Example 17, the following compounds were prepared.

### 2-[3-(4-Methoxyphenyl)propionylamino]benzamide (Compound 72)

¹H-NMR (CDCl₃, 400MHz) δ ppm:
   2.69 (t, J=7.8Hz, 2H), 3.01 (t, J=7.8Hz, 2H), 3.78 (s, 3H), 5.4-6.5 (br, 2H), 6.75-7.6 (m, 7H), 8.65 (dd, J=8.8, 1.1 Hz, 1H), 11.12 (s, 1H)

### 2-[3-(3,4,5-Trimethoxyphenyl)propionylamino]benzamide (Compound 73)

¹H-NMR (DMSO-*d*_{*6*}, 400MHz) δ ppm:
   2.6-2.95 (m, 4H), 3.60 (s, 3H), 3.73 (s, 6H), 6.57 (s, 2H), 7.0-7.95 (m, 4H), 8.25 (brs, 1H), 8.46 (d, J=8.2Hz, 1H), 11.70 (s, 1H)

### Example 19

### 2-(3-Ethoxyphenylacetylamino)benzamide (Compound 74)

To 3-ethoxyphenylacetic acid (500 mg) were added thionyl chloride (5 ml) and a drop of *N*,*N*-dimethylformamide, and the mixture was stirred at 80 °C for 30 minutes. Toluene was added to the reaction mixture, and the mixture was concentrated under reduced pressure. The residue was dissolved in tetrahydrofuran (10 ml), and 2-aminobenzamide (830 mg) was added to the solution under ice-cooling, and the mixture was stirred for 20 minutes at room temperature. To the reaction mixture was added water, and the mixture was extracted with ethyl acetate. The extract was washed with 1 N hydrochloric acid, an aqueous saturated sodium hydrogen carbonate solution and brine successively, dried over anhydrous magnesium sulfate and concentrated under reduced pressure to give 2-(3-ethoxyphenylacetylamino)benzamide (780 mg).
¹H-NMR (DMSO-*d*_{*6*}, 400MHz) δ ppm:
   1.32 (t, J=7.0Hz, 3H), 3.64 (s, 2H), 4.01 (q, J=7.0Hz, 2H), 6.7-7.8 (m, 8H), 8.23 (s, 1H), 8.44 (dd, J=8.4, 1.0Hz, 1H), 11.74 (s, 1H)

### Example 20

### 2-(4-Chlorophenylacetylamino)benzamide (Compound 75)

To a suspension of 4-chlorophenylacetic acid (500 mg) in toluene (5 ml) were added thionyl chloride (3 ml) and a drop of *N*,*N*-dimethylformamide, and the mixture was stirred at 80 °C for 30 minutes. The reaction mixture was concentrated under reduced pressure, and the residue was dissolved in tetrahydrofuran (10 ml). To the solution was added 2-aminobenzamide (840 mg) under ice-cooling, and the mixture was stirred for 5 minutes at room temperature. To the reaction mixture was added water, and the mixture was extracted with ethyl acetate. The extract was washed with 1 N hydrochloric acid, an aqueous saturated sodium hydrogen carbonate solution and brine successively, dried over anhydrous magnesium sulfate and concentrated under reduced pressure to give 2-(4-chlorophenylacetylamino)benzamide (807 mg).
¹H-NMR (DMSO-*d*_{*6*}, 400MHz) δ ppm:
   3.72 (s, 2H), 7.0-7.85 (m, 8H), 8.23 (brs, 1H), 8.42 (dd, J=8.4, 1.0Hz, 1H), 11.72 (s, 1H)

### Example 21

In the similar manner to that in Examples 19 and 20, the following compounds were prepared. 2-(3,4-Dimethoxyphenylacetylamino)benzamide (Compound 76)
¹H-NMR (CDCl₃, 400MHz) δ ppm:
   3.68 (s, 2H), 3.86 (s, 3H), 3.89 (s, 3H), 5.9-6.3 (br, 2H), 6.8-7.1 (m, 4H), 7.4-7.6 (m, 2H), 8.66 (d, J=8.4 Hz, 1H), 11.19 (s, 1H)

### 2-(4-Ethoxyphenylacetylamino)benzamide (Compound 77)

¹H-NMR (DMSO-*d*_{*6*}, 400MHz) δ ppm:
   1.31 (t, J=7.0Hz, 3H), 3.60 (s, 2H), 3.99 (q, J=7.0Hz, 2H), 6.87 (d, J=8.7Hz, 2H), 7.0-7.8 (m, 6H), 8.22 (brs, 1H), 8.44 (dd, J=8.4, 1.0Hz, 1H), 11.71 (s, 1H)

### 2-(2-Methoxyphenylacetylamino)benzamide (Compound 78)

¹H-NMR (CDCl₃, 400MHz) δ ppm:
   3.75 (s, 2H), 3.86 (s, 3H), 5.2-6.4 (br, 2H), 6.8-7.6 (m, 7H), 8.5-8.7 (m, 1H), 10.87 (brs, 1H)

### 2-(2,5-Dimethoxyphenylacetylamino)benzamide (Compound 79)

¹H-NMR (CDCl₃, 400MHz) δ ppm:
   3.72 (s, 2H), 3.79 (s, 3H), 3.82 (s, 3H), 5.3-6.3 (br, 2H), 6.8-7.55 (m, 6H), 8.61 (dd, J=8.8, 1.1Hz, 1H), 10.90 (brs, 1H)

### 2-(3-Methoxyphenylacetylamino)benzamide (Compound 80)

¹H-NMR (CDCl₃, 400MHz) δ ppm:
   3.71 (s, 2H), 3.82 (s, 3H), 5.7-6.5 (br, 2H), 6.8-7.6 (m, 7H), 8.63 (dd, J=8.8, 1.1Hz, 1H), 11.18 (brs, 1H)

### 2-(3,5-Dimethoxyphenylacetylamino)benzamide (Compound 81)

¹H-NMR (CDCl₃, 400MHz) δ ppm:
   3.67 (s, 2H), 3.80 (s, 6H), 5.6-6.3 (br, 2H), 6.40 (t, J=2.2Hz, 1H), 6.54 (d, J=2.2Hz, 2H), 7.0-7.55 (m, 3H), 8.6-8.7 (m, 1H), 11.19 (brs, 1H)

### 2-(3,4,5-Trimethoxyphenylacetylamino)benzamide (Compound 82)

¹H-NMR (DMSO-*d*_{*6*}, 400MHz) δ ppm:
   3.62 (s, 2H), 3.63 (s, 3H), 3.78 (s, 6H), 6.67 (s, 2H), 7.0-7.8 (m, 4H), 8.21 (brs, 1H), 8.43 (dd, J=8.0, 1.0Hz, 1H), 11.63 (s, 1H)

### 2-(2-Chlorophenylacetylamino)benzamide (Compound 83)

¹H-NMR (DMSO-*d*_{*6*}, 400MHz) δ ppm:
   3.85 (s, 2H), 7.0-7.85 (m, 8H), 8.21 (brs, 1H), 8.46 (dd, J=8.4, 0.9Hz, 1H), 11.74 (s, 1H)

### 2-(3,4-Methylenedioxyphenylacetylamino)benzamide (Compound 84)

¹H-NMR (DMSO-d₆, 400MHz) δ ppm:
   3.60 (s, 2H), 5.99 (s, 2H), 6.7-8.5 (m, 9H), 11.67 (s, 1H)

### 2-(4-Ethoxy-3-methoxyphenylacetylamino)benzamide (Compound 85)

¹H-NMR (CDCl₃, 400MHz) δ ppm:
   1.42 (t, J=6.9Hz, 3H), 3.69 (s, 2H), 3.88 (s, 3H), 4.08 (q, J=6.9Hz, 2H), 6.2-7.7 (m, 8H), 8.69 (d, J=8.7Hz, 1H), 11.20 (s, 1H)

### Example 22

### 2-(3-Ethoxybenzoylamino)benzamide (Compound 86)

To 3-ethoxybenzoic acid (600 mg) were added thionyl chloride (6 ml) and a drop of *N*,*N*-dimethylformamide, and the mixture was stirred at 80 °C for 30 minutes. Toluene was added to the reaction mixture, and the resulting mixture was concentrated under reduced pressure. The residue was dissolved in pyridine (6 ml) under ice-cooling, and 2-aminobenzamide (540 mg) was added to the solution. The mixture was stirred at 80°C for 10 minutes and then at 130 °C for 30 minutes. To the reaction mixture was added water, and the mixture was extracted with ethyl acetate. The extract was washed with 1 N hydrochloric acid, an aqueous saturated sodium hydrogen carbonate solution and brine successively, dried over anhydrous magnesium sulfate and concentrated under reduced pressure to give 2-(3-ethoxybenzoylamino)benzamide (917 mg).
¹H-NMR (DMSO-*d*_{*6*}, 400MHz) δ ppm:
   1.37 (t, J=7.0Hz, 3H), 4.11 (q, J=7.0Hz, 2H), 7.1-8.05 (m, 8H), 8.43 (brs, 1H), 8.70 (dd, J=8.4, 1.1Hz, 1H), 12.96 (s, 1H)

### Example 23

In the similar manner to that in Example 22, the following compounds were prepared.

### 2-(3,5-Dimethoxybenzoylamino)benzamide (Compound 87)

¹H-NMR (DMSO-*d*_{*6*}, 400MHz) δ ppm:
   3.83 (s, 6H), 6.76 (t, J=2.2Hz, 1H), 7.08 (d, J=2.2Hz, 2H), 7.15-8.0 (m, 4H), 8.43 (brs, 1H), 8.69 (dd, J=8.4, 1.1Hz, 1H), 12.96 (s, 1H)

### 2-(3,4-Dimethoxybenzoylamino)benzamide (Compound 88)

¹H-NMR (DMSO-*d*_{*6*}, 270MHz) δ ppm:
   3.85 (s, 6H), 7.1-8.5 (m, 8H), 8.70 (d, J=8.1Hz, 1H), 12.90 (s, 1H)

### 2-(3,4,5-Trimethoxybenzoylamino)benzamide (Compound 89)

¹H-NMR (DMSO-*d*_{*6*}, 400MHz) δ ppm:
   3.76 (s, 3H), 3.88 (s, 6H), 7.1-8.05 (m, 6H), 8.45 (brs, 1H), 8.70 (d, J=8.4Hz, 1H), 13.04 (s, 1H)

### Example 24

### (E)-2-(3,4-Dimethoxycinnamoylamino)-5-hydroxybenzamide (Compound 90)

To a saturated ammonia solution (25 ml) of methyl (*E*)-2-(3,4-dimethoxycinnamoylamino)-5-hydroxybenzoate (100 mg) in methanol was added sodium cyanide (1.4 mg), and the mixture was heated in a sealed tube at 40 °C for 2 days. The solvent was removed under reduced pressure, and separation and purification of the residue by column chromatography on silica gel (eluent: chloroform/ methanol = 10/1) gave (*E*)-2-(3,4-dimethoxycinnamoylamino)-5-hydroxybenzamide (28 mg).
¹H-NMR (DMSO-*d*_{*6*}, 400MHz) δ ppm:
   3.79 (s, 3H), 3.83 (s, 3H), 6.68 (d, J=15.6Hz, 1H), 6.85-7.25 (m, 4H), 7.33 (d, J=2.0Hz, 1H), 7.47 (d, J=15.6Hz, 1H), 7.57 (brs, 1H), 8.12 (brs, 1H), 8.27 (d, J=9.0Hz, 1H), 9.4-9.65 (br, 1H), 11.11 (s, 1H)

### Example 25

### (E)-2-(3,4-Dimethoxycinnamoylamino)-N-(2-dimethylaminoethyl)benzamide hydrochloride (Compound 91)

To a solution of (*E*)-2-(3,4-dimethoxycinnamoylamino)-*N*-(2-dimethylaminoethyl)benzamide (100 mg) in ethanol (1 ml) was added 1 N hydrochloric acid (1 ml), and the mixture was stirred for 5 minutes at room temperature. The reaction mixture was concentrated under reduced pressure to give (*E*)-2-(3,4-dimethoxycinnamoylamino)-*N*-(2-dimethylaminoethyl)benzamide hydrochloride (109 mg).
¹H-NMR (CDCl₃, 400MHz) δ ppm:
   2.88 (s, 3H), 2.90 (s, 3H), 3.25-3.35 (m, 2H), 3.80-4.00 (m, 8H), 6.48 (d, J=15.5Hz, 1H), 6.87 (d, J=8.3Hz, 1H), 7.05-7.20 (m, 3H), 7.45-7.55 (m, 1H), 7.67 (d, J=15.5Hz, 1H), 8.09 (dd, J=8.0, 1.3Hz, 1H), 8.70-8.80 (m, 1H), 8.98 (t, J=5.6Hz, 1H), 11.60(s, 1H), 12.10-12.25 (br, 1H)

### Example 26

In the similar manner to that in Example 25, the following compounds were prepared.

### (E)-N-(2-Dimethylaminoethyl)-2-(4-ethoxy-3-methoxycinnamoylamino)benzamide hydrochloride (Compound 92)

¹H-NMR (DMSO-*d*_{*6*}, 400MHz) δ ppm:
   1.33 (t, J=7.0Hz, 3H), 2.83 (s, 3H), 2.84 (s, 3H), 3.2-3.75 (m, 4H), 3.82 (s, 3H), 4.05 (q, J=7.0Hz, 2H), 6.74 (d, J=15.5Hz, 1H), 6.9-9.1 (m, 9H), 9.95-10.15 (br, 1H), 11.27 (s, 1H)

### (E)-N-(2-Dimethylaminoethyl)-2-(3,4,5-trimethoxycinnamoylamino)benzamide hydrochloride (Compound 93)

¹H-NMR (DMSO-*d*_{*6*}, 400MHz) δ ppm:
   2.83 (s, 3H), 2.84 (s, 3H), 3.15-3.75 (m, 7H), 3.84 (s, 6H), 6.86 (d, J=15.6Hz, 1H), 7.0-7.25 (m, 3H), 7.5-9.15 (m, 5H), 10.05-10.25 (br, 1H), 11.30 (s, 1H)

### Example 27

### Inhibition test on vascular smooth muscle cells proliferation

Spontaneous hypertensive rat-derived vascular smooth muscle cells (VSMCs) in thoracic aorta were isolated by explant technique. The VSMCs were placed into 96 wells at 5000 cells/well and incubated in DMEM containing 10 % fetal calf serum at 37 °C in 95 % air and 5 % carbon dioxide for 3 days. After the incubation, the medium was replaced with DMEM containing each compound and ³H-thymidine, and then the VSMCs were incubated. 24 hours later, the incorporation of ³H-thymidine into DNA was determined and the radioactivity was defined as proliferation inhibitory activity of VSMCs. The activity of each compound was shown as a concentration which reduced the radioactivity of non-treated group by 50 % (IC₅₀). The results are summarized in the following table.

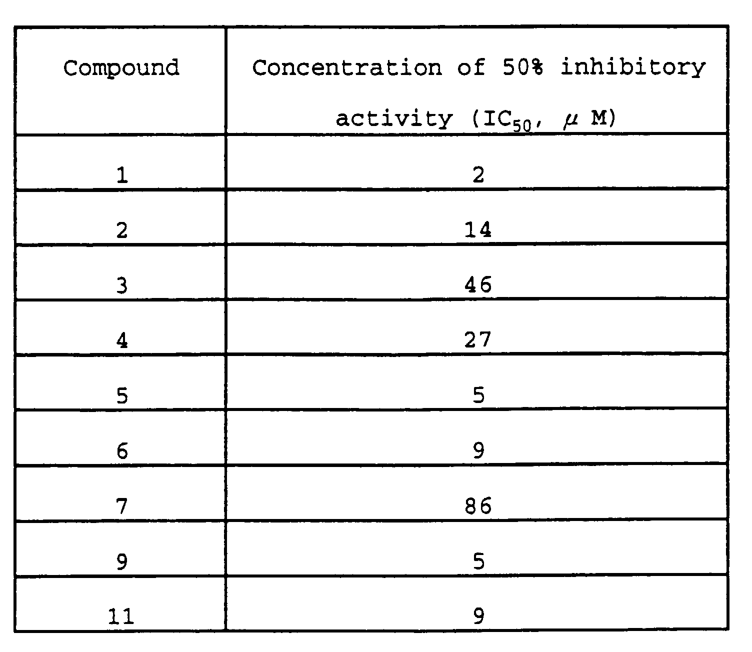

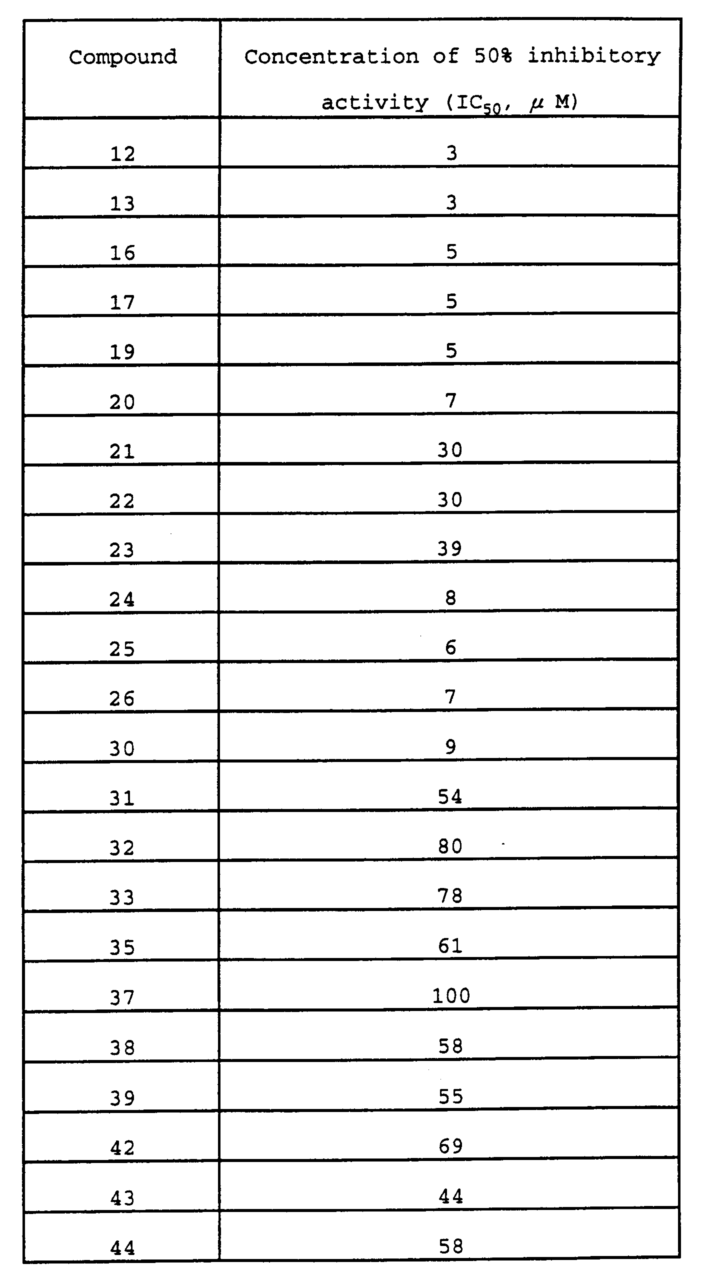

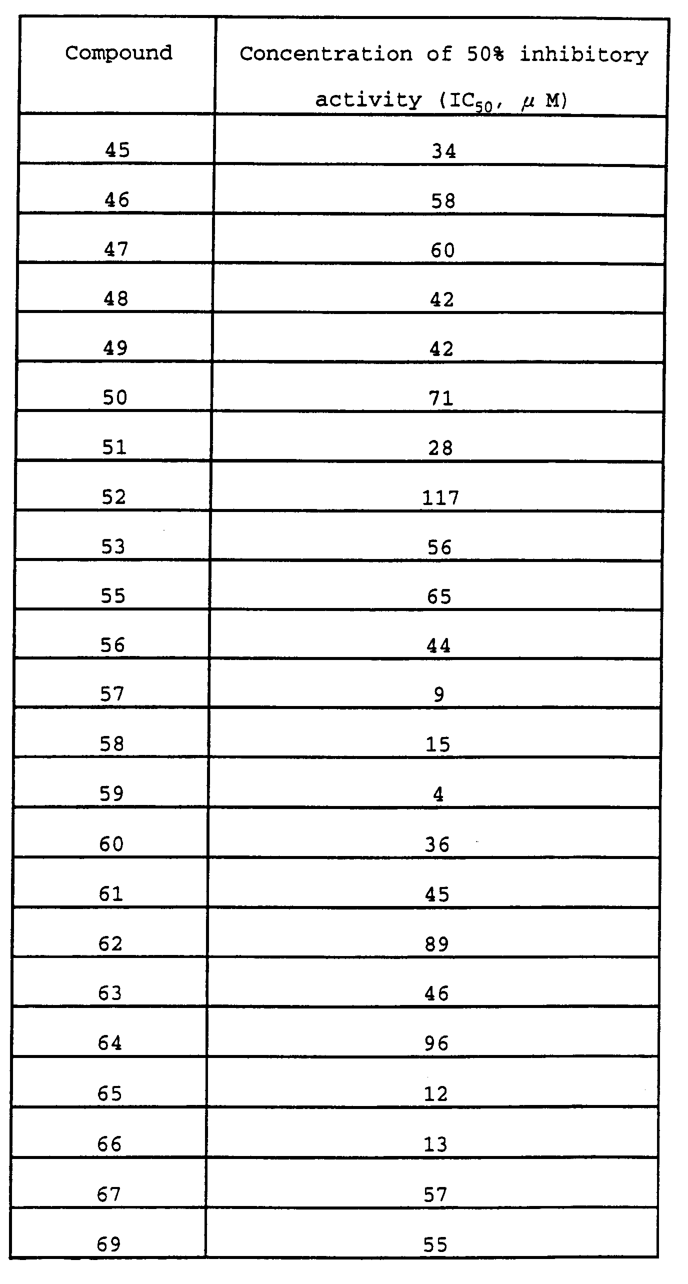

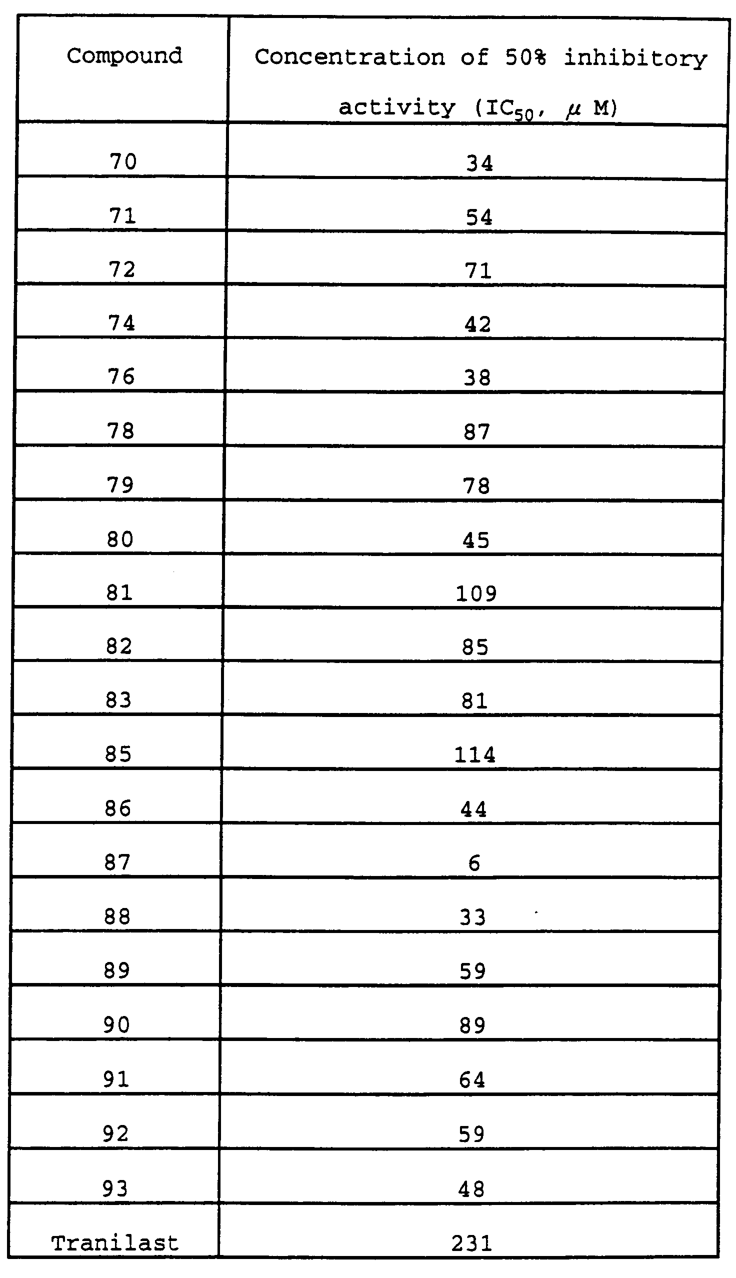

### Example 28

### Inhibition test on neointimal hyperplasia

The effect of compounds *in vivo* was evaluated by using a model of thrombosis-induced neointimal hyperplasia in mouse femoral artery. Each compound was suspended in 0.5 % carboxymethylcellulose sodium solution and administered orally to mice once a day for 3 weeks. After final administration, the femoral artery was fixed and removed. Cross sections of vessels were made consecutively and the neointimal area induced and formed by thrombosis was measured. Inhibitory activity on neointimal hyperplasia of each compound was shown as a dose which reduced the area of the non-treated group by 50 % (ID₅₀). The results are summarized in the following table.

| Compound | Dose of 50% inhibitory activity (ID₅₀, mg/kg) |
|---|---|
| 4 | 1 |
| 17 | 1 |
| Tranilast | 100 |

### Example 29

### Acute toxicity test

Male ICR nice were fasted for 4 hours. 2-(3,4-Dimethoxycinnamoylamino)benzamide was suspended in 0.5 % carboxymethylcellulose sodium and administered orally at doses of 300, 1000 and 2000mg/kg, respectively. Dead mice and abnormal behavior were not observed even in the group administered at a dose of 2000mg/kg.

### Preparation 1

| Fine granules | |
|---|---|
| Active compound | 100mg |
| Lactose | 600mg |
| Corn starch | 265mg |
| Hydroxypropylcellulose | 30mg |
| Calcium stearate | 5mg |
| | (Total 1000mg) |

### Preparation 2

| Capsules | |
|---|---|
| Active compound | 100mg |
| Crystalline cellulose | 50mg |
| Carmellose calcium | 13mg |
| Hydroxypropylcellulose | 4mg |
| Calcium stearate | 3mg |
| | (Total 170mg) |

## Claims

1. An agent for the prevention and treatment of diseases caused by excessive proliferation of vascular intimal cells which comprises, as an active ingredient, a 2-acylaminobenzamide derivative represented by the general formula: 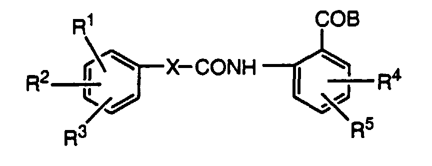 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which may be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X represents a vinylene group which may have a cyano group, an acetylene group, an ethylene group, a methylene group or a single bond; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group; a group represented by the general formula:
-NH-(CH₂)ₙ-A-R⁸
wherein A represents a single bond, a group represented by the general formula:
-O-(CH₂)ₘ-
wherein m in an integer of from 2 to 6 or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6 ; R⁸ represents a hydroxy group or an amino group which nay be mono- or di-substituted by lower alkyl groups; and n is an integer of from 2 to 6 or a hydroxyamino group or a pharmaceutically acceptable salt thereof.

2. An agent for the prevention and treatment of diseases caused by excessive proliferation of vascular intimal cells as claimed in claim 1, which comprises, as an active ingredient, a 2-acylaminobenzamide derivative represented by the general formula: 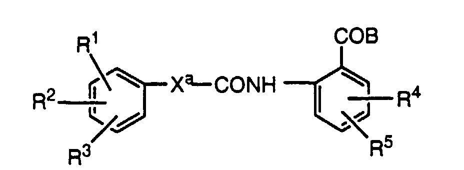 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which may be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X^{a} represents a vinylene group or a methylene group; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group, a group represented by the general formula:
-NH-(CH₂)ₙ-A-R⁸
wherein A represents a single bond, a group represented by the general formula:
-O-(CH₂)ₘ-
wherein m in an integer of from 2 to 6 or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6; R⁸ represents a hydroxy group or an amino group which may be mono- or di-substituted by lower alkyl groups; and n is an integer of from 2 to 6 or a hydroxyamino group or a pharmaceutically acceptable salt thereof.

3. An agent for the prevention and treatment of diseases caused by excessive proliferation of vascular intimal cells as claimed in claim 1, which comprises, as an active ingredient, a 2-acylaminobenzamide derivative represented by the general formula: 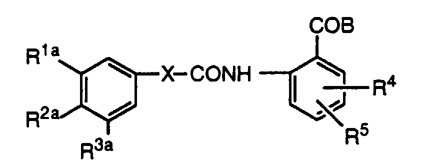 wherein R^{1a} represents a hydrogen atom or a lower alkoxy group; R^{2a} and R^{3a} are the same or different and each represents a lower alkoxy group; X represents a vinylene group which may have a cyano group, an acetylene group, an ethylene group, a methylene group or a single bond; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group), a group represented by the general formula:
-NH-(CH₂)ₙ-A-R⁸
wherein A represents a single bond, a group represented by the general formula:
-O-(CH₂)ₘ-
wherein m in an integer of from 2 to 6 or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6; R⁸ represents a hydroxy group or an amino group which may be mono- or di-substituted by lower alkyl groups; and n is an integer of from 2 to 6; or a hydroxyamino group or a pharmaceutically acceptable salt thereof.

4. An agent for the prevention and treatment of diseases caused by excessive proliferation of vascular intimal cells as claimed in claim 3, which comprises, as an active ingredient, a 2-acylaminobenzamide derivative represented by the general formula: 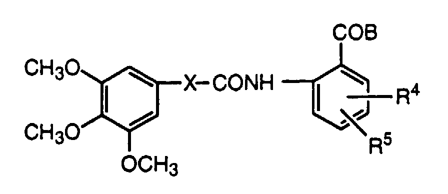 wherein X represents a vinylene group which may have a cyano group, an acetylene group, an ethylene group, a methylene group or a single bond; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group, a group represented by the general formula:
-NH-(CH₂)ₙ-A-R⁸
wherein A represents a single bond, a group represented by the general formula:
-O-(CH₂)ₘ-
wherein m in an integer of from 2 to 6 or a group represented by the general formula:
-N(R⁹)(CH₂)ₚ-
wherein R⁹ represents a hydrogen atom or a lower alkyl group which nay have a hydroxy group, an amino group or a mono or di(lower alkyl)amino group as a substituent; and p is an integer of from 2 to 6; R⁸ represents a hydroxy group or an amino group which may be mono- or di-substituted by lower alkyl groups; and n is an integer of from 2 to 6 or a hydroxyamino group or a pharmaceutically acceptable salt thereof.

5. An agent for the prevention and treatment of diseases caused by excessive proliferation of vascular intimal cells as claimed in claim 1, which comprises, as an active ingredient, a 2-acylaminobenzamide derivative represented by the general formula: 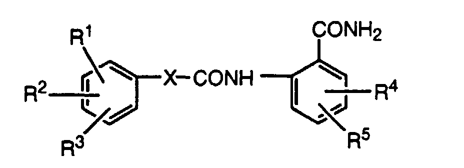 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which may be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X represents a vinylene group which nay have a cyano group, an acetylene group, an ethylene group, a methylene group or a single bond; and R⁴ and R⁵ are the same or different and each represents a hydrogen atom, a hydroxy group, a halogen atom, a lower alkyl group or a lower alkoxy group or a pharmaceutically acceptable salt thereof.

6. An agent for the prevention and treatment of diseases caused by excessive proliferation of vascular intimal cells as claimed in claim 1, which comprises, as an active ingredient, a 2-acylaminobenzamide derivative represented by the general formula: 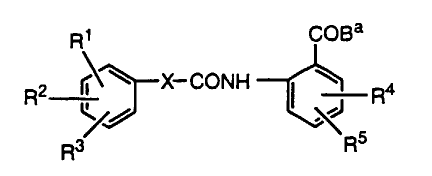 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which nay be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X represents a vinylene group which may have a cyano group, an acetylene group, an ethylene group, a methylene group or a single bond; R⁴ and R⁵ are the same or different and each represents a hydrogen atom, a hydroxy group, a halogen atom, a lower alkyl group or a lower alkoxy group; B^{a} represents a group represented by the general formula:
-NH-(CH₂)ₙ-A-R⁸
wherein A represents a single bond, a group represented by the general formula:
-O-(CH₂)ₘ-
wherein m in an integer of from 2 to 6 or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6 . R⁸ represents a hydroxy group or an amino group which may be mono- or di-substituted by lower alkyl groups; and n is an integer of from 2 to 6 or a pharmaceutically acceptable salt thereof.

7. The agent for the prevention and treatment of diseases caused by excessive proliferation of vascular intimal cells as claimed in claims 2, 4 or 5, which comprises, as an active ingredient, the 2-acylaminobenzamide derivative represented by the formula:

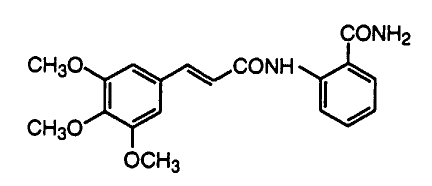

8. The agent for the prevention and treatment of diseases caused by excessive proliferation of vascular intimal cells as claimed in claims 2, 4 or 6, which comprises, as an active ingredient, the 2-acylaminobenzamide derivative represented by the formula:

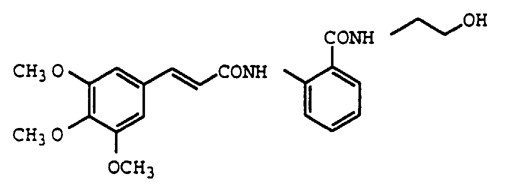

9. The agent for the prevention and treatment of diseases caused by excessive proliferation of vascular intimal cells as claimed in claims 2, 4 or 6, which comprises, as an active ingredient, the 2-acylaminobenzamide derivative represented by the formula: 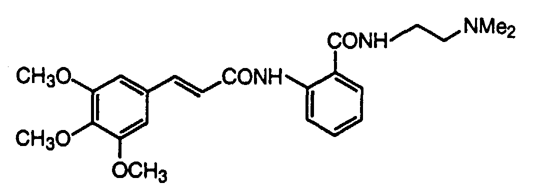 or pharmaceutically acceptable salt thereof.

10. A pharmaceutical composition comprising a 2-acylaminobenzamide derivative represented by the general formula: 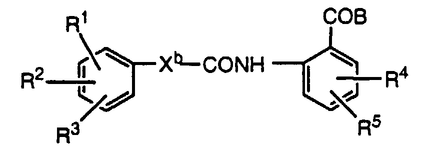 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which may be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X^{b} represents a vinylene group which may have a cyano group, an acetylene group, an ethylene group or a methylene group; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group), a group represented by the general formula:
-NH-(CH₂)ₙ-A-R⁸
wherein A represents a single bond, a group represented by the general formula:
-O-(CH₂)ₘ-
wherein m in an integer of from 2 to 6 or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6; R⁸ represents a hydroxy group or an amino group which nay be mono- or di-substituted by lower alkyl groups; and n is an integer of from 2 to 6 or a hydroxyamino group; with the proviso that X^{b} dose not represent a methylene group and R⁶ and R⁷ dose not represent a hydrogen atom when R¹, R² and R³ represent a hydrogen atom at the same time; and with the proviso that X^{b} dose not represent a vinylene group, n is not 2 or 3, A dose not represent a single bond and R⁸ dose not represent a dimethylamino group or a diethylamino group when R¹, R² and R³ represent a hydrogen atom at the same time or a pharmaceutically acceptable salt thereof.

11. A 2-acylaminobenzamide derivative represented by the general formula: 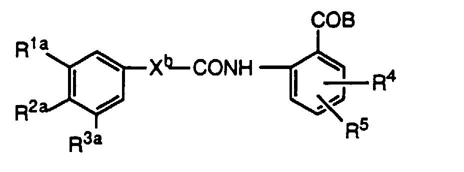 wherein R^{1a}, R^{2a} and R^{3a} are the same or different and each represents a lower alkoxy group; X^{b} represents a vinylene group which may have a cyano group, an acetylene group, an ethylene group or a methylene group; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group, a group represented by the general formula:
-NH-(CH₂)ₙ-A-R⁸
wherein A represents a single bond, a group represented by the general formula:
-O-(CH₂)ₘ-
wherein m in an integer of from 2 to 6) or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6; R⁸ represents a hydroxy group or an amino group which may be mono- or di-substituted by lower alkyl groups; and n is an integer of from 2 to 6 or a hydroxyamino group] and a pharmaceutically acceptable salt thereof.

12. A 2-acylaminobenzamide derivative as claimed in claim 11, represented by the general formula: 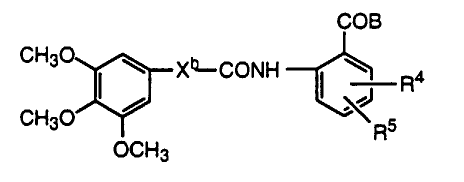 wherein X^{b} represents a vinylene group which may have a cyano group, an acetylene group, an ethylene group or a methylene group; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group a group represented by the general formula:
-NH-(CH₂)ₙ-A-R⁸
wherein A represents a single bond, a group represented by the general formula:
-O-(CH₂)ₘ-
wherein m in an integer of from 2 to 6 or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6; R⁸ represents a hydroxy group or an amino group which may be mono- or di-substituted by lower alkyl groups; and n is an integer of from 2 to 6] or a hydroxyamino group and a pharmaceutically acceptable salt thereof.

13. A 2-acylaminobenzamide derivative represented by the general formula: 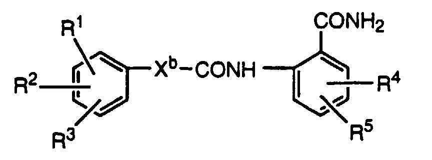 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which nay be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X^{b} represents a vinylene group which may have a cyano group, an acetylene group, an ethylene group or a methylene group; R⁴ and R⁵ are the same or different and each represents a hydrogen atom, a hydroxy group, a halogen atom, a lower alkyl group or a lower alkoxy group; and with the proviso that when one of R¹, R² and R³ represents a hydrogen atom, the others does not represent a hydrogen atom or a methoxy group at the same time and a pharmaceutically acceptable salt thereof.

14. A 2-acylaminobenzamide derivative represented by the general formula: 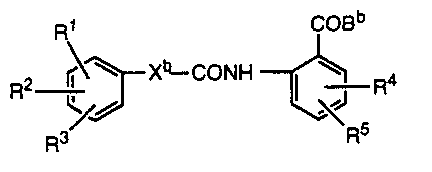 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which may be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X^{b} represents a vinylene group which may have a cyano group, an acetylene group, an ethylene group or a methylene group; R⁴ and R⁵ are the same or different and each represents a hydrogen atom, a hydroxy group, a halogen atom, a lower alkyl group or a lower alkoxy group; B^{b} represents a group represented by the general formula:
-NH-(CH₂)ₙ-A-R⁸
wherein A represents a single bond, a group represented by the general formula:
-O-(CH₂)ₘ-
wherein m is an integer of from 2 to 6) or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6; R⁸ represents a hydroxy group or an amino group which may be mono- or di-substituted by lower alkyl groups; and n is an integer of from 2 to 6 or a hydroxyamino group; and with the proviso that n is not 2 or 3, A does not represent a single bond and R⁸ does not rerpesent a dimethylamino group or a diethylamino group when R¹, R² and R³ represent a hydrogen atom at the same time and X^{b} represents a vinylene group and a pharmaceutically acceptable salt thereof.

15. A 2-acylaminobenzamide derivative as claimed in claims 12 or 13, represented by the formula:

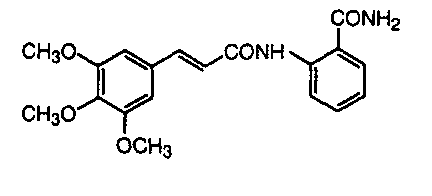

16. A 2-acylaminobenzamide derivative as claimed in claims 12 or 14, represented by the formula:

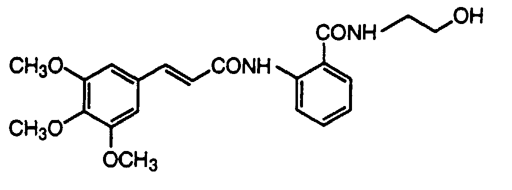

17. A 2-acylaminobenzamide derivative as claimed in claims 12 or 14, represented by the formula: 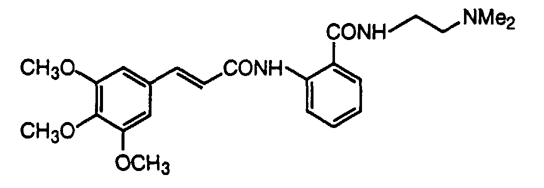 and pharmaceutically acceptable salt thereof.

18. A method for the prevention and treatment of diseases caused by excessive proliferation of vascular intimal cells which comprises administering a 2-acylaminobenzamide derivative represented by the general formula: 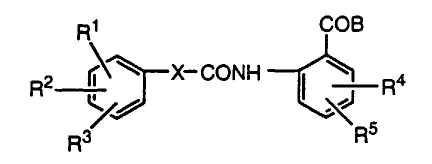 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which nay be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X represents a vinylene group which may have a cyano group, an acetylene group, an ethylene group, a methylene group or a single bond; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group, a group represented by the general formula:
-NH-(CH₂)ₙ-A-R⁸
wherein A represents a single bond, a group represented by the general formula:
-O(CH₂)ₘ-
wherein m in an integer of from 2 to 6) or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6; R⁸ represents a hydroxy group or an amino group which may be mono- or di-substituted by lower alkyl groups; and n is an integer of from 2 to 6 or a hydroxyamino group or a pharmaceutically acceptable salt thereof.

19. A use of a 2-acylaminobenzamide derivative represented by the general formula: 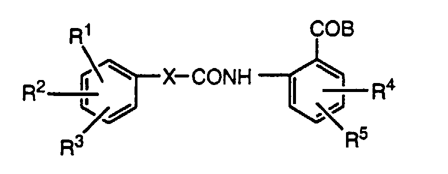 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which may be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X represents a vinylene group which may have a cyano group, an acetylene group, an ethylene group, a methylene group or a single bond; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group , a group represented by the general formula:
-NH-(CH₂)ₙ-A-R⁸
wherein A represents a single bond, a group represented by the general formula:
-O-(CH₂)ₘ-
wherein m in an integer of from 2 to 6) or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6); R⁸ represents a hydroxy group or an amino group which may be mono- or di-substituted by lower alkyl groups; and n is an integer of from 2 to 6 or a hydroxyamino group or a pharmaceutically acceptable salt thereof for the manufacture of a pharmaceutical composition for the prevention and treatment of diseases caused by excessive proliferation of vascular intimal cells.

20. A use of a 2-acylaminobenzamide derivative represented by the general formula: 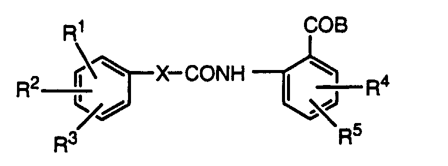 wherein R¹ represents a hydrogen atom, a halogen atom, a hydroxy group, a lower alkyl group, a lower alkoxy group, a hydroxy lower alkoxy group, a cycloalkylalkoxy group, an aralkyloxy group, a lower acyl group, a nitro group, a cyano group, an amino group which may be mono- or di-substituted by lower alkyl groups, a carboxyl group, a lower alkoxycarbonyl group or a lower alkylsulfonyl group; R² and R³ are the same or different and each represents a hydrogen atom or a lower alkoxy group, or R² and R³ are adjacent to each other and together form a lower alkylene group containing oxygen atoms in the chain; X represents a vinylene group which may have a cyano group, an acetylene group, an ethylene group, a methylene group or a single bond; R⁴ and R⁵ are the same or different and each represents a hydrogen atom, a hydroxy group, a halogen atom, a lower alkyl group or a lower alkoxy group; B represents a group represented by the general formula:
-N(R⁶)(R⁷)
wherein R⁶ and R⁷ are the same or different and each represents a hydrogen atom, a lower alkyl group, a cycloalkylalkyl group or an aralkyl group), a group represented by the general formula:
-NH-(CH₂)ₙ-A-R⁸
wherein A represents a single bond, a group represented by the general formula:
-O-(CH₂)ₘ-
wherein m in an integer of from 2 to 6) or a group represented by the general formula:
-N(R⁹)(CH₂)ₚ-
wherein R⁹ represents a hydrogen atom or a lower alkyl group which may have a hydroxy group, an amino group or a mono or di(lower alkyl)amino group as a substituent; and p is an integer of from 2 to 6 ; R⁸ represents a hydroxy group or an amino group which may be mono- or di-substituted by lower alkyl groups; and n is an integer of from 2 to 6 or a hydroxyamino group or a pharmaceutically acceptable salt thereof as an agent for the prevention and treatment of diseases caused by excessive proliferation of vascular intimal cells.
